(19) Europäisches Patentamt
European Patent Office
Office européen des brevets

(11) EP 2 176 654 B1

(12) **EUROPEAN PATENT SPECIFICATION**

(45) Date of publication and mention of the grant of the patent:
**22.03.2017 Bulletin 2017/12**

(21) Application number: **08776089.8**

(22) Date of filing: **30.07.2008**

(51) Int Cl.:
***G01N 33/543*** *(2006.01)*

(86) International application number:
**PCT/GB2008/002591**

(87) International publication number:
**WO 2009/016366 (05.02.2009 Gazette 2009/06)**

(54) **BIOSENSOR**

BIOSENSOR

BIODÉTECTEUR

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **31.07.2007 GB 0714866**

(43) Date of publication of application:
**21.04.2010 Bulletin 2010/16**

(73) Proprietor: **University Of Leeds**
**Leeds**
**West Yorkshire LS2 9JT (GB)**

(72) Inventors:
- **NELSON, Laurence, Andrew**
  **Leeds LS7 4LN (GB)**
- **COLDRICK, Zachary**
  **Leeds LS6 4RQ (GB)**

(74) Representative: **Atkinson, Peter Birch et al**
**Marks & Clerk LLP**
**1 New York Street**
**Manchester M1 4HD (GB)**

(56) References cited:
**US-A- 5 378 343 US-A1- 2001 045 359**

- **ZAWISZA I. ET. AL.: "Electrochemistry of Langmuir-Blodgett and sefl-organized monolayers of an azocrown ether, both pure and mixed with a phospholipid" JOURNAL OF ELECTROANALYTICAL CHEMISTRY, vol. 509, 2001, pages 31-41, XP002501008**
- **NELSON A ET AL: "Interaction of hydrophobic organic compounds with mercury adsorbed dioleoylphosphatidylcholine monolayers" BIOCHIMICA ET BIOPHYSICA ACTA. BIOMEMBRANES, AMSTERDAM, NL, vol. 1021, no. 2, 29 January 1990 (1990-01-29), pages 205-216, XP023352574 ISSN: 0005-2736 [retrieved on 1990-01-29] cited in the application**
- **KOUNAVES S P ET AL: "IRRIDIUM-BASED ULTRAMICROELECTRODE ARRAY FABRICATED BY MICROLITHOGRAPHY" ANALYTICAL CHEMISTRY, AMERICAN CHEMICAL SOCIETY. COLUMBUS, US, vol. 66, no. 3, 1 February 1994 (1994-02-01), pages 418-423, XP000434607 ISSN: 0003-2700**
- **ECONOMOU A ET AL: "Applications, potentialities and limitations of adsorptive stripping analysis on mercury film electrodes" TRAC, TRENDS IN ANALYTICAL CHEMISTRY, ELSEVIER, AMSTERDAM, NL, vol. 16, no. 5, 1 May 1997 (1997-05-01), pages 286-292, XP004097044 ISSN: 0165-9936**
- **MAUZEROLL J ET AL: "SCANNING ELECTROCHEMICAL MICROSCOPY. 48. HG/PT HEMISPHERICAL ULTRAMICROELECTRODES: FABRICATION AND CHARACTERIZATION", ANALYTICAL CHEMISTRY, AMERICAN CHEMICAL SOCIETY, US, vol. 75, no. 15, 1 August 2003 (2003-08-01), pages 3880-3889, XP001175989, ISSN: 0003-2700, DOI: 10.1021/AC034088L**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 176 654 B1

## Description

The present invention relates to an electrode assembly having an electrode incorporating a phospholipid layer simulating a biomembrane, to an electrochemical biosensor incorporating such an assembly, and to the use of the biosensor. The sensor may be used, for example, for electrochemically analysing a sample to determine the presence (or otherwise) of a species having biomembrane activity (e.g. a toxin) or for investigating whether a species (e.g. a potential pharmaceutical) has biomembrane activity.

Surface layers on electrodes have been studied for some time as membrane models for electrochemical interrogation[1]. One method has exploited the fact that long chain alkanethiols can be bound to metallic surfaces such as gold or silver using the thiol linkage to form self assembled layers (SAM)[2]. The system is employed as a biosensor, for example by looking at analyte specific binding reactions on the film surface[3] or by investigating coupled enzyme reactions with faradaic species in solution[4]. The advantages of this approach for sensing applications are the stability of the system. A disadvantage is that the bound layers are not fluid and do not entirely resemble a cell membrane. Because of this disadvantage, the system has been elaborated by deposition of phospholipid layers non-covalently on the tethered layer to make an asymmetrical bilayer[5]. However, the complexity of the resulting model membrane system leads away from the simplicity required for a robust analytical device. An alternative strategy for the study of surface membrane like layers on electrodes has been the non-covalent deposition of surfactant/phospholipid films on electrode surfaces[6-19]. An advantage of this approach is that the layers are mobile, undergo reversible phase transitions as a function of applied potential and desorb at extreme potentials (> 1 *V*)[10,17,18]. These processes underlie the mechanisms involved in the electroporation of bilayers and biological membranes[19].

Relevant in this regard is GB-A-2 193 326 (Natural Environment Research Council) which discloses a biosensor based on the use of a hanging drop mercury electrode (HDME) in which each successive mercury drop is formed with a phospholipid layer for the purpose of a measurement to be made with the electrode. More specifically, the HMDE (which functions as a working electrode in an electrochemical cell further comprising an auxiliary electrode and a reference electrode) has a capillary tip (at which successive mercury drops may be formed) which is reciprocally moveable into and out of an electrolyte liquid having on its surface a film of a phospholipid (e.g. dioleoyl lecithin or egg lecithin). A species to be investigated by the biosensor is included in the electrolyte liquid. In one cycle of operation, a mercury drop is formed at the tip whilst it is within the liquid and below the lipid film. The tip is then withdrawn upwardly through the film and then downwardly back into the liquid. In this way, there is formed on the surface of the mercury drop a lipid film which has a very similar structure and properties to half a biological membrane. Measurements to investigate the species in solution may then be made by monitoring the phospholipid layer on the mercury electrode immersed in the electrolyte liquid by rapid cyclic voltammetry using a saw tooth waveform at a rapid ramp rate (e.g. 40 V $s^{-1}$). At low voltages, the resulting current is proportional to the capacitance of the surface of the electrode at high voltages, the capacitance shows sharp peaks, representing phase changes of the phospholipid layer which correspond to its fluidity and (given that the electrolyte liquid does not contain any biomembrance active components) is very characteristic of the pure phospholipid. These peaks are shown for dioleoyl phosphatidylcholine (DOPC) in accompanying Fig 6a (see below). However the presence of a species having biomembrane activity changes the fluidity and affects the phase changes, thus influencing the form of the peaks. The biosensor may therefore be used for detecting the presence of biomembrane active components in a sample. The detection limit depends on the nature of the particular compound in the sample but for some polyaromatic compounds is in the region of 1 ppb in water.

The system of phospholipid monolayers non covalently deposited on liquid mercury thus represents a unique and biologically relevant case due to the compatibility of the fluid phospholipid with the liquid mercury. Three features emerge from this property:

(i) The phase transitions representing the ingression of water and subsequent orientational changes are sharp and are represented by distinct capacitative peaks in a cyclic and *ac* voltammetry plot[9].

(ii) The phospholipid layer on mercury is sensitive to interaction with biological membrane active species which change the structure and fluidity or organisation of the layer in a selective manner. The interactions influence the nature of the phase transitions and the impedance properties of the layer[14]. These interactions are different from those commonly exploited in membrane based biosensors which rely on a binding reaction between an analyte species and moeities attached to the membrane or monolayer/bilayer[16].

Recently we showed that the phospholipid-Hg system could be also used to screen peptides in solution and that it responded selectively to the functionality of short peptides[14]. Initially the interaction between the layers and gramicidin derivatives was examined[11,12], later studies looked at the modification of the monolayer system by an anti-microbial peptide[21,22] and finally the relationship between the structure and the monolayer activity of short chain eleven residue $\beta$-sheet self assembling peptides was examined[14]. In all cases the interactions mirrored very closely the biological

membrane activity of the peptides.

In spite of the distinct advantages discussed above, the phospholipid/hanging Hg drop electrode (HMDE) system has numerous severe drawbacks. In particular the HMDE is fragile so that the HMDE system is most suitable for use in a laboratory so that application "in the field" is limited. Additionally, the HMDE requires the use of relatively large amounts of liquid Hg with consequential toxicity factors to be taken into consideration. Moreover the HMDE can only be imaged with difficulty [24].

It is therefore an object of the present invention to obviate or mitigate the above mentioned disadvantage.

According to a first aspect of the present invention there is provided an electrode assembly as defined in claim 1.

According to a second aspect of the invention there is provided a biosensor comprising

(i) an electrode assembly as defined for the first aspect of the invention,
(ii) at least one counter electrode for the working electrodes,
(iii) a reference electrode
(iv) means for applying a periodically varying voltage to the at least one working electrode, and
(v) means for determining variations in the differential capacitance of the phospholipid against the counter electrode.

Therefore in the electrode assembly of the first aspect of the invention, the working electrode is comprised of metallic (liquid) mercury (on which the phospholipid layer is provided) immobilised on a Pt conductive substrate. Electrodes comprised of metallic mercury deposited on a conductive substrate are known in the art and are often referred to as "thin film mercury electrodes" and sometimes as "amalgam electrodes". Thus, for example, mercury-on-iridium electrodes are known and are disclosed, for example, in US-A-5 378 343 (Kounaves). This prior specification discloses an electrode assembly format comprising an array of ultramicroelectrodes arranged on a substrate, each ultramicroelectrode comprising mercury-on-iridium. The electrodes disclosed in US-A-5 378 343 are proposed for use in detecting various heavy metals in water by means of anodic stripping voltammetry in which, initially, a negative potential is applied to the mercury so that metal ions in solution are electrochemically reduced and concentrated into the mercury and, subsequently, the applied potential is scanned slowly in the positive direction which results in a peak current at the oxidation potential of each metal proportional to its concentration. There is however no disclosure in US-A-5 378 343 of coating the mercury with a phospholipid for the purpose of investigating species having biomembrane activity.

Document J. Electroanal. Chem., vol. 509 (2001) 31-41 describes an electrode assembly according to the preamble of claim 1.

Electrode assemblies in accordance with the first aspect of the invention have a number of advantages. In particular, the electrode assembly is robust (in contrast to the somewhat fragile nature of the hanging mercury drop electrode) thus allowing a biosensor incorporating such an electrode assembly to have a wide range of uses outside the laboratory. The immobilised phospholipid layer can give rise to sharper peaks than for a hanging drop mercury electrode system, the sharper peaks being better for analytical purposes. Additionally the amount of mercury required for an electrode can be significantly decreased as compared to a hanging drop mercury electrode, thus significantly reducing toxicity. Furthermore, there are advantages (as compared to a hanging mercury drop electrode) in relation to the stability of the phospholipid layer which is dependent on the ratio of edge to surface area. In the case of an electrode assembly in accordance with the invention, the ratio of edge to surface area of the working electrode can be very much larger than in the case of a mercury drop so that the stability of the phospholipid layer on the surface will be higher. In this regard we have demonstrated (see Example 6 below) that electrode assemblies in accordance with the invention (i.e. with phospholipid deposited on the mercury coating) may be subjected to repeated interrogation by cyclic voltammetry and produce reproducible results over significant periods of time. Additionally, the phospholipid layer may be cleaned off the mercury coating and a fresh layer applied. In a series of tests (see also Example 6 below) we have established that (for a particular composite electrode comprised of the conducting substrate with mercury coating) the successively deposited phospholipid layers provide a high degree of reproducibility in terms of the results obtained by interrogating the layers by cyclic voltammetry.

Conveniently, the cleaning of the phospholipid from the mercury surface may be effected by scanning the working electrode in a cathodic direction (e.g. over the range (-0.2 V to -2.625 V) @ 97 $Vs^{-1}$) with the electrode being immersed in electrolyte so as to desorb any contaminating organic material into the bulk solution. Surprisingly we have found that similar scan conditions when applied for much shortly periods of time than used for cleaning can be used to deposit the phospholipid layer on the mercury film. For the purposes of this deposition, the electrode will be immersed in electrolyte which incorporates the phospholipid to be deposited. The phospholipid may be added to the aqueous electrolyte in the form of a dispersion prepared by agitation (e.g. using sonication) of the phospholipid in an aqueous medium. During the deposition procedure, the mercury electrode may be monitored by cyclic voltammetry and deposition of the layer may be detected by the appearance in a cyclic voltammogram of a trace characteristic of the phospholipid. With the appearance of this trace, the deposition procedure will be complete.

Electrode assemblies in accordance with the invention provide results very similar to those obtained for the Hanging Mercury Drop Electrode of the prior art. Consequently much of the extensively recorded data for the HMDE may be applied to electrode assemblies in accordance with the invention. However in contrast to the HMDE, the mercury surface of the composite electrode (i.e. the electrode comprised of the conductive substrate and the mercury coating) may be re-used in a repetitive cycle of steps (i) to (iii) below:

(i) depositing a fresh phospholipid layer;
(ii) effecting a measurement on a sample; and
(iii) cleaning the electrode to remove the "used " phospholipid.

Consequently there is no need to regenerate the mercury coating for each measurement, cf the HMDE for which a mercury drop is generated *in situ* for each successive measurement.

Significantly we have established that the mercury layer (even though it is in the form of a liquid immobilised on the surface) has sufficient adhesion to the conductive substrate on which it is deposited to allow the electrode assembly to be used in a flow cell as described more fully herein. In combination with the features described above, such a flow cell may be operated in a repeated series of steps which comprise:

(i) deposition of phospholipid on to the mercury coating (with electrolyte flowing through the cell);
(ii) effecting a measurement on a sample passed through the cell; and
(iii) cleaning the phospholipid layer from the mercury coating.

It will be appreciated that operation of such a flow cell may be easily automated and provides a very convenient measurement technique.

The electrode assembly in accordance with the invention is conveniently provided on a "chip" assembly which also incorporates a working electrode and a reference electrode. Such a chip assembly is eminently suitable for use in the above described flow cell. Embodiments of the invention are therefore able to provide a "lab-on-$\alpha$-chip" functionally suitable for on-line measurement purposes.

We believe platinum has a particularly appropriate solubility with respect to mercury to allow production of an "amalgam-like" joint which holds the mercury relatively strongly on the platinum whilst providing a good surface for the mercury to allow phospholipid deposition and provide good membrane activity. Furthermore, in construction of electrode assemblies in accordance with the invention on a "chip" (e.g. based on a silicon wafer) the use of platinum as the conductive carrier substrate allows a reference electrode to be incorporated readily on the same chip.

The biosensor of the second aspect of the invention functions by monitoring the lipid layer and in particular the modification thereof due to the presence in a sample under investigation of a species having biomembrane activity. Measurements are made by voltammetry to determine variations in the differential capacitance of the phospholipid as a function of voltage against the reference electrode, in a similar manner to that disclosed in GB-B 2 193 326. Most preferably measurement is by means of rapid cyclic voltammetry, preferably using a sawtooth waveform with a ramp rate of $\geq 1\ Vs^{-1}$ (e.g. 40-100 $Vs^{-1}$). The voltage excursion used in rapid cyclic voltammetry may be from -0.4 V to -1.2 V vs Ag/AgCl 3.5 M KC1. The output current (i) is proportional to the differential capacitance ($C_d$) as indicated by the equation:

$$C_d = i / (\nu \text{ x } A)$$

where A is the electrode area and *v* is the ramp rate.

The experimental set-up for RCV involves the application of the sawtooth wave form using a function generator with input to a potentiostat which applies the waveform to the working electrode. The resulting current response is recorded via an acquisition board and plotted against the applied waveform.

DC cyclic voltammetry provides rapid assessment of the layer's capacity over a defined potential window specific to the phospholipid monolayer's structure and environment.

Alternatively measurement may be made by ac voltammetry using, for example, a voltage ramp of about 5 mV $s^{-1}$ with a superimposed sinusoidal voltage of frequency, f, about 75 Hertz and of amplitude, $\Delta E$, about 0.005 V. The output ac current is separated into both in phase and out of phase components. The out of phase current (i") is proportional to the differential capacitance ($C_d$) as expressed by the equation:

$$C_d = i'' / (2\pi \text{ x } f \text{ x } \Delta E \text{ x } A)$$

The experimental set-up for ac voltammetry involves adding the sinusoidal waveform to the above voltage ramp and inputting to a potentiostat which applies the resulting waveform to the working electrode. The ac current response is fed into a lock-in amplifier where the in phase and out of phase components with the applied ac waveform of the current are separated and recorded on a data acquisition system. The out of phase current is plotted against the ramp voltage.

The electrode assembly of the first aspect of the invention may comprise a single working electrode but more preferably comprises a plurality of the working electrodes. For the or each working electrode there should be no exposed free conductive carrier substrate surface. This ensures that instability issues associated with hydrogen gas production *via* water reduction during voltammetry measurements with the working electrode in contact with an aqueous media are circumvented by the large hydrogen overpotential provided by the mercury layer.

The working electrode may for example be circular and/or have a maximum surface dimension in any direction of 2$\mu$m to 1mm, although dimensions outside this range are not precluded. The Examples described below utilise a circular electrode having a diameter of about 960 $\mu$m. Alternatively, the working electrode may be a microelectrode and preferably sized such that the mercury has a maximum surface dimensions of 2$\mu$m to 10$\mu$m in any direction. The or each working electrode may, for example, be circular with a diameter of 2$\mu$m to 10$\mu$m. Such dimensions serve to maximise the edge-to area and thereby enhance stability of the phospholipid layer.

Preferably the electrode assembly comprises a layer of a conductive carrier substrate of platinum, sandwiched between first and second insulating substrate layers (preferably silica), one of which (the "first" substrate layer) is penetrated by at least one through aperture which, in effect, defines a well for which said carrier metal provides a basal surface, the well incorporating the mercury coating (for said carrier metal) on which the phospholipid layer is provided, thereby forming a said working electrode. In the case of a microelectrode, the or each well may, for example, be circular and have a selected diameter in the range 2$\mu$m to 10$\mu$m and (given that the mercury layer is hemispherical) may have a depth of about 1 $\mu$m which corresponds therefore to the wall thickness.

The mercury layer and its phospholipid coating should occupy the full cross-section of the well (so no carrier substrate is exposed). This total coverage of the carrier substrate at the basal surface of the well suppresses water reduction by carrier substrate during the voltammetry measurement. Ideally also the configuration of the mercury layer and its phospholipid coating are such that the latter has a planar surface flush with the surface of the first insulating substrate layer.

A preferred microelectrode array in accordance with the invention comprises a plurality of said wells formed in the first insulator layer, the carrier substrate at the base of each of said wells being coated with mercury which in turn is provided with the lipid layer. Such a microelectrode array thus comprises a carrier metal layer provided between the insulating substrates with portions of the carrier substrate providing respective basal surfaces for the individual wells.

Conveniently the electrode assembly may incorporate a conducting layer (e.g. gold) sandwiched between the second insulating substrate and the carrier metal layer with which the conducting layer is in electrically conducting relationship, thereby improving the conductivity of the device.

Microelectrode arrays in accordance with the invention may incorporate electrodes additional to the "mercury-on-carrier metal" electrodes. Thus, for example, the array may incorporate the reference electrode (e.g. Ag/AgCl) / pseudo-reference (e.g. Pt) and/or the counter electrode (e.g Pt) provided on an exterior surface of the first insulting substrate.

Microelectrode arrays in accordance with the invention comprise:

(i) first and second insulating layers,
(ii) a layer of a carrier metal of platinum,
(iii) a plurality of wells formed in the first layer such that the carrier metal layer provides respective basal surfaces for the wells, said discrete portions each forming part of a working electrode comprised of said discrete portion, a mercury coating therefor and a phospholipid layer on the surface of the mercury,
(iv) optionally a conducting layer provided between said carrier metal layer and the second substrate and being in electrically conducting relationship therewith,
(v) a counter electrode provided on the first insulating layer, and
(vi) a reference electrode provided on the first insulating layer.

The first insulating layer may, for example, be silica or silicon nitride.

The second insulating may be silica and may be formed on a silicon wafer.

In a preferred microelectrode array in accordance with the invention, the carrier metal is platinum which, in addition to providing the surface on which the mercury is deposited, also serves to provide conductive traces connecting the electrode of the invention to electrical contact means provided on the array. In this embodiment, the separate conducting layer (iv) may be omitted.

Preferably the wells are circular, e.g. with a diameter of 2-10$\mu$m although diameters outside this range may be

used, the reference electrode is Ag/Ag Cl and/or the counter electrode is platinum.

A microelectrode array comprised of (i)-(vi) defined above is able to provide a so-called "lab-on-$\alpha$-chip" functionality suitable for on-line use.

Electrode assemblies in accordance with the invention may be used in a static cell or a flow-cell. Such a flow cell may comprise a "measurement cell" (e.g. in the form of a chamber) in which the working electrodes are exposed. The flow-cell will further comprise inlet and outlet channels communicating with the "measurement cell". In use of the flow-cell electrolyte and analyte will be supplied *via* the inlet channel(s) to the "measurement cell" where they flow over the working electrode and then out through the outlet channel(s). There may be more than one type of working electrode (of the invention) in the "measurement cell", with the various electrodes being distinguished by the particular phospholipid deposited on the mercury surface. Each electrode of the flow cell may be connected (e.g. by an appropriate mechanical or electronic switching arrangement) potentiostat such that each working electrode may be addressed individually. Thus individual results can be obtained for the various different phospholipids. Alternatively the combined signal from the various electrode assemblies (with their different phospholipids) may be recorded. In these ways, the effect of the same analyte on a plurality of different phospholipids may be determined and provide a "fingerprint" for that analyte.

The phospholipid provided as a layer on the surface of the mercury may be saturated or may have a degree of unsaturation. Examples of suitable phospholipids include dimyristoyl, phosphatidyl choline (DMPC - saturated), dioleoyl phosphhtidyl choline (DOPC - unsaturated) and egg lecithin (egg PC - saturated/unsaturated) Such lipids incorporate choline-based head groups and other lipids incorporating this head group may be used. However the head group may be amine based as in dioleoyl phosphatidylethanolamine (DOPE) or hydroxyl based as in 1,2-dioleoyl-sn-glycero-3-phospho(ethylene glycol) (sodium salt) or contain a combination of chemical functional groups as in 1,2-dioleoyl-sn-glycero-3-phosphoethanolamine-N-(succinyl) (sodium salt). These functional groups may be present in 'natural' lipids or may be synthesized to confer a desired chemical surface. Each lipid present as a homogeneous monolayer or as a fraction of a heterogeneous monolayer will confer unique properties to that layer that may be observed through the layers capacitance over the interrogation potential window. Modification of the phospholipid head group may be used and be important in the sensor's operation due to this being the functionalised surface that is presented directly to the electrolyte. The layers properties may be tuned by changing the length of the carbon chains and their saturation or through the modification of the head groups.

More specifically, phospholipids with two 9-cis-octadecenoic chains are fluid at room temperature and capable of forming impermeable monolayers on mercury. They exhibit sharp pseudo capacitative phase transitions within the potential range interrogated by rapid cyclic voltammetry. By comparison, dimyristoyl lipids (incorporating $C_{14}$ saturated chains) have the advantage of being less susceptible to oxidation than dioleoyl lipids but exhibit less prominent phase transitions.

If desired the phospholipid layer may be associated with additional components for modifying the properties of that layer. These components may be incorporated within the layer or covalently tethered thereto and examples include peptides (to form ion channels), oligonucleotides or molecules complementary to the target molecule.

Microelectrode arrays in accordance with the invention may be produced by successively depositing on to an insulating substrate (which ultimately provides the aforementioned second insulating substrate) an optional conducting layer (e.g. gold) and a carrier metal layer (e.g. both by E-beam evaporation) prior to deposition of a further insulating layer (the aforementioned first layer) to overlie the carrier metal. The second insulating layer, may, for example, be a silicon wafer with a $SiO_2$ surface layer. Deposition of the optional conducting layer (e.g. gold) and carrier metal layer may for example be E-beam evaporation. If desired, titanium adhesion layers may be deposited between (i) the second substrate and the conducting layer, (ii) between the conducting layer and the carrier metal layer, and (iii) on to the carrier metal layer. The first insulating layer may, for example be $SiO_2$ or silicon nitride and deposited by low temperature plasma enhanced chemical vapour deposition (PECVD). Subsequently the first insulating layer may be etched through to the carrier metal so that the aforementioned wells are formed with the carrier metal providing a basal surface for the wells.

If desired, reference and/or counter electrodes may be formed on the first layer prior to the next step of the fabrication procedure in which mercury is deposited on the carrier metal.

The mercury layer may be formed by eletrodeposition. The amount of mercury deposited should be sufficient to give a continuous film of the mercury on the conductive carrier substrate. Increasing the thickness of the mercury layer will enhance the stability of that layer and allow repeated the mercury layer to allow repeated cycles of phospholipid layer deposition, sample measurement and removal of the phospholipid layer so that one electrode may be used many times without disruption of the mercury. Additionally thickness of mercury layer will be a consideration for use of the electrode assembly in a flow cell where the mercury layer is required to withstand forces associated with liquid flows through the cell. The amount of charge required for the electrodeposition process will depend on the required thickness of the mercury layer and this will in turn depend on factors such as the surface area of the conductive substrate (on to which the mercury is to be electrodeposited) and the concentration of mercury ions in the mercury deposition electrolyte. By way of example, Example 4 uses one Coulomb of charge to deposit a satisfactory mercury layer on to a circular platinum substrate having a diameter of 960$\mu$m at the particular mercury concentration in the deposition electrolyte.

Example 7 below (illustrating use of a flow cell) employs an electrode formed by using two Coulombs of charge to deposit mercury on to a circular platinum substrate having a diameter of 960$\mu$nm. For the same concentration of solution, lesser amounts of charge will be required for platinum substrates of smaller diameter. The converse is true for substrates of larger diameter.

Controlled electrodeposition of mercury from a solution containing $Hg^{2+}$ ions (e.g. provided by $Hg(NO_3)_2$) may be effected within an electrochemical cell. The mercury deposition electrolyte may contain $HClO_4$ which prevents oxidation of the Hg species. However the lipid interrogation will not take place in this acidic solution and will instead be in a more benign electrolyte such as 0.1M KCl. Alternatively electrodeposition of mercury can be effected by pipetting a drop of the base electrolyte (e.g. $HClO_4$ + Hg(II)) onto the surface of the first insulator. The electrodeposition may be facilitated by applying a potential of ~0.4V vs. 3.5 M KCl Ag/AgCl to the working electrode surface in the deposition solution and recording the charge passed which is directly proportional to the mass of mercury deposited the deposition can be stopped by breaking the circuit. This allows precise control over the amount of Hg deposited.

Once the mercury layer has been deposited, the assembly can be transferred to a neutral electrolyte such as 0.1M KCl and the electrode surface electrochemically cleaned by applying an extreme negative potential <-2V evolving hydrogen gas to 'scrub' the surface free from organics.

Subsequently the phospholipid may be deposited on the mercury. This may be effected in a number of ways, for example:

(a) The deposition can be carried out as has been done previously with the HMDE by transferring the phospholipid on to the array from an excess of the phospholipid provided as a film on a liquid. Both vertical and horizontal insertion through the solution gas interface can be effected. This procedure leads to formation of the phospholipid as a monolayer on the mercury surface.

(b) A controlled deposition can be carried out in a Langmuir Blodgett trough by inserting the array vertically through a phospholipid monolayer of precise coverage at the solution gas interface. Advantage is taken of the controlled deposition by calibrating the properties of the phospholipid layers with the phospholipid coverage at the gas-solution interface. As with (a), a monolayer of the phospholipid is formed on the mercury surface.

(c) A phospholipid layer may be deposited onto the array surface by allowing the evaporation of a concentrated pentane solution containing the phospholipid of interest subsequent to the array being immersed in the solution. The layer may then be made homogeneous by submerging the array in an aqueous electrolyte such as 0.1M KCl and rapidly cycling the potential from -0.2 to -1.2V continuously. This method provides for formation of a phospholipid monolayer by facilitating self-assembly through applying a rapid potential ramp which causes the phospholipids on the surface to continuously rearrange themselves into the lowest energy configuration (ie: a monolayer at -0.4V vs 3.5M KCl Ag/AgCl which is close to the Potential of Zero Charge (PZC) of the mercury).

(d) A drop of phospholipid can be evaporated on the silicon wafer. When free of solvent the wafer is transferred to an electrochemical cell. In this case a monolayer is obtained on the electrode array by continued scanning using RCV. This allows the phospholipids to anneal into a monolayer.

(e) A vesicle or phospholipid dispersion can be allowed to adsorb during flow inside a flow cell. As with (d), a monolayer is obtained by continued scanning using RCV, whilst the phospholipid solution is flowed over the electrode array.

Using the above methods of deposition the configuration of the phospholipid on the surface can be evaluated using RCV, ACV and impedance together with atomic force microscopy (AFM) studies. In RCV and ACV the characteristics of the two capacitance peaks, corresponding to the phospholipid phase transitions determine the configuration and coverage of the phospholipid layer on the electrode[20]. The voltammograms of the deposited phopholipid can be checked to gauge if the voltammometric peaks representing the phospholipid phase transitions are influenced by electrode size. Indeed both unsaturated (eg DOPC), saturated (eg DMPC) and saturated/unsaturated (eg egg lecithin (egg PC)) phospholipids can be tested, to evaluate their stability in the presence of air. The phospholipid layer may be removed by desorption from the electrode surface at the extreme potential of -2.0 vs 3.5M KCl Ag/AgCl. The mercury surface will be routinely cleaned in this manner.

The biosensor of the invention may be used, for example, for determining (i) the presence or otherwise in a sample of a species known to have biomembrane activity or (ii) whether or not a particular substance has biomembrane activity. The measurements may be made by voltammetry, (e.g. rapid cyclic voltammetry or ac-voltammetry effected in conventional manner such as along the lines disclosed in GB-A-2 193 326 (see for example Fig 1 thereof). RCV will be used for rapid interrogation of the sensor surface and in particular to determine the effect of the interaction of the two

capacitance of the phospolipid which are particular sensitive to interactions.

Generally the determination procedure will involve an aqueous electrolyte liquid in which the electrode assembly in an electrochemical cell will be immersed, if necessary, electrolytes (e.g. KCl) can be added to the liquid. It is however also contemplated that one or more drops of the electrolyte liquid could be applied to electrode assembly for the purposes of the measurement without the need for locating the assembly in an electrochemical cell. In this case it will be necessary to use phospholipids which are stable in the presence of air. Saturated dimyristoyl phosphatidylcholine,(DMPC) has been shown to be suitable for this and it also displays potential induced phase transitions which can be used for analytical applications. Also within the scope of the invention is the analysis of gas or vapour samples for the presence therein of a species with biomembrane activity. In this case it would be necessary for an electrolyte liquid to be provided on the working electrode.

The invention has applications in a wide number of fields. These include:

(a) routine testing of drinking water for the presence of biomembrane active compounds which may be toxic agents;
(b) testing of potential pharmaceutical products for their biomembrane activity;
(c) detection of toxic gases and explosive vapours which (provided they interact with the monolayer surface) may be diagnosed using a multivariate analysis approach depending on the strength of the interaction with monolayers of varying chemical functionality; and
(d) environmental applications such as the *in situ* analysis of natural and marine waters for biomembrane active compounds such as (i) toxic biomembrane active peptides produced by blooms of cyanobacteria and dinoflagelates or (ii) pollutants.

It will be appreciated that the system may be calibrated for sensor application with a series of compounds eg polycyclic aromatic hydrocarbons[7], phenothiazine drugs[8] and anti-microbial peptides[23] which are known to modify the structure of phospholipid layers on electrodes. In each case the disruption/modification of the phospholipid layer has been well characterised electrochemically.

In the case of a microelectrode array, the individual working electrodes may incorporate phospholipids varying in the functionality of lipid head groups that respond in a specific, but different, fashion to target analytes. The chemical nature of the target analyte can be dissected by multi-variant analysis of the magnitudes of interactions with the array monolayer surface presented functional groups. Phospholipids with two 9-cis-octadecenoic chains are 18 carbon atoms long, unsaturated and fluid at room temperature capable of forming semi-permeable monolayers on mercury. They exhibit sharp pseudo capacitative phase transitions within the potential region interrogated by rapid CV. Also of interest are dimyristoyl lipids which are 14 carbon atom saturated chains that has the advantage of being less susceptible to oxidation than di-oleoyl lipids but exhibit less prominent phase transitions. The sensor can employ a combination of these lipids with varying functional head groups so that each array possesses a single homogenous population of phospholipids presenting a functionalised surface with which the target analyte can interact. The phospholipids acting as a transducer to the interaction allow for further incorporation of selective elements, either peptide based such as immunoglobins, oligo-nucleotides or complementary molecules to the target molecule either incorporated with-in the layer or covalently tethered to the layer.

The invention will be further described by way of example only with reference to the accompanying drawings, in which:

Fig 1 is a schematic cross-section (to a much enlarged scale) of one embodiment of electrode assembly not in accordance with the invention;

Fig 2 is a perspective view of an intermediate structure for the fabrication of an electrode assembly in accordance with the invention;

Fig 3 (a)-(i) illustrate steps in the production of a microelectrode array of the type for which Fig 2 shows an intermediate structure;

Fig 4 illustrates a further embodiment of microelectrode array in accordance with the invention;

Fig 5 schematically illustrates the principal components of a measurement system incorporating the microelectrode array of Fig 4;

Fig 6 demonstrates the results of Example 1, more particularly experimental results for a biosensor in accordance with the invention in comparison with those for a hanging drop mercury electrode arrangement of the prior art;

Fig 7 demonstrates the results of Example 2, more particularly experimental results for a microelectrode array in accordance with the invention;

Fig 8 is a schematic cross-section (to a much enlarged scale) of a further embodiment of electrode assembly in accordance with the invention;

Fig 9 is a schematic perspective view (to a much enlarged scale) of a further embodiment of electrode assembly in accordance with the invention;

Figs 10(a)-(c) are (to a much enlarged scale) schematic sectional, end and plan views respectively of one embodiment of flow cell in accordance with the invention;

Figs 11(a) and 11(b) are cyclic votltammetry scans obtained for a composite electrode comprising platinum with a mercury coating;

Fig 13 is a cyclic voltammetry scan for a Hanging Mercury Drop Electrode;

Fig 14 illustrates the chemical formulae of various phospholipids;

Figs 15(a)-(e) are specific capacitance plots for electrode assemblies in accordance with the invention incorporating the phospholipids illustrated in Fig 14;

Fig 16 (a)-(c) are specific capacitance plots demonstrating stability of electrode assemblies in accordance with the invention as carried out in Example 6;

Figs 17 and 18 illustrate results obtained using a flow cell in accordance with the procedure of Example 7;

Figs 19(a) and (b) are specific capacitance plots for a Hanging Mercury Drop Electrode and Pt/Hg electrode as obtained following the procedure of Example 8;

Figs 20(a) and (b) show forward and reverse scans comparing respectively a HMDE electrode and an electrode in accordance with the invention, both incorporating a monolayer film of DOPC; and

Figs 21 to 28 illustrate the results obtained in Examples 10 to 17 respectively each of which compares results obtained for a HMDE and an electrode assembly in accordance with the invention for analysis of specific analytes and/or amounts thereof.

Referring firstly to Fig 1, an electrode assembly 1 not in accordance with the invention comprises a working electrode 2 which is comprised of the combination of an iridium layer 3 having a surface coating of mercury 4 on which is deposited a phospolipid monolayer 5.

As illustrated in Fig 1, the mercury layer 4 is located within a well structure (for which iridium layer 3 provides a basal surface) formed in an upper silica layer 6, the well being circular with a selected diameter in the range 2μm to 10μm and having a depth of 0.5μm. The mercury occupies the full cross-section of the well structure whereby there is no exposed free iridium.

Additional principal features of the illustrated electrode assembly are a lower silicon substrate 7 with a silica surface and a gold conducting layer 8 which is a electrically conducting relationship with iridium layer 3. Provided between the silicon/silica layer 7 and gold layer 8 is a titanium adhesion layer 9 and similar adhesion layers 10 and 11 are provided respectively

(i) between the iridium layer 3 and gold layer 8, and
(ii) between iridium layer 3 and upper insulating layer 6.

Reference is now made to Fig 2 which is a perspective view of a microelectrode array based on the structure illustrated in Fig 1 but with the titanium adhesion layers 9-11 omitted for the purposes of clarity and the well structures (referenced in Fig 2 as 12) being shown as "empty" (i.e. without the mercury layer 4 and its associated phospholipid monolayer 5). Fig 2 does however also illustrate circular and part-circular reference electrodes 13 provided on the upper insulating layer 6, each electrode 13 having a radius of 50μm and each being centred at a respective well 12.

Fig 3 illustrates a step wise procedure for producing an electrode assembly of the type illustrated in Fig 2.

The illustrated procedure starts with a 10cm x 10cm silicon wafer with a 90nm $SiO_2$ surface layer (e.g. as available from IDB Technologies). This provides layer 7 for the structure illustrated in Figs 1 and 2.

There is then deposited in succession on to the $SiO_2$ surface layer (a) the titanium adhesion layer 9 (30nm), (b) the gold conduction layer 8 (100nm), (c) the titanium adhesion layer 10 (30nm), (d) the iridium layer 3 (30nm) and (e) the titanium adhesion layer 11 (30nm). All of these layers may be deposited by E-beam evaporation. It should be noted that, at this stage, the titanium adhesion layer 11 is deposited as a continuous layer. The resulting structure is depicted in Fig 3a.

In the next step of the process, a 500nm $SiO_2$ layer is deposited on titanium adhesion layer 11 by means of low-temperature plasma enhanced chemical vapour deposition (PECVD) to provide layer 6 (but not having, at this stage, the wells 12 formed therein). The product at this stage is shown in Fig $_{3b}$.

For the next step of the process, a positive resist material is spun on to the $SiO_2$ layer 6 then baked at 150°C and washed in chlorobenzene to provide a "hardened" surface resist layer depicted by reference numeral 14 (see Fig 3c).

A mask 15 incorporating the pattern for the silver rings 13 is positioned over resist layer 14 which is then patterned using photo/E-beam lithography (Fig 3(d)).

Mask 15 is now removed and next resist layer 14 is chemically etched down to the $SiO_2$ layer 6 (Fig 3e).

In the step illustrated in Fig 3(f) a 100nm silver layer 16 is evaporated on to the surface of the resist layer 14 but, more importantly, also within the channels of the pattern of circles and arcs defined therein.

Subsequently resist layer 14 is removed to leave a structure as illustrated in Fig 3(g) in which the upper surface has circular and part circular traces 17.

Although not illustrated in the drawings, procedures generally along the lines described for Figs 3 (c)-(f) may be repeated to provide a platinum counter electrode on the silica layer 6.

A further resist layer 18 capable of withstanding plasma etching is now applied to silica layer 6 (and overlays the silver traces 17) and plasma etched to form a pattern of circular apertures 19, that expose the iridium layer (Fig 3(h). In other words, the etching is through the $SiO_2$ layer 6 and also through the titanium adhesion layer 11.

If desired, a 10nm gold wetting layer may be deposited at this stage to the exposed iridium surfaces at the bases of the apertures 19.

Removal of the resist 18 produces the structure illustrated in Fig 3(i) in which wells 12 (corresponding to those in Fig 2) have been formed.

The silver trace 12 may be anodised in a chloride rich solution to produce a stable Ag/Ag/Cl reference electrode with fast kinetics and a stable reference potential.

The layer of mercury 4 (described with reference to Fig 1) may be electrodeposited (as described above) on to those portions of the iridium layer 3 exposed at the base of the wells. Subsequently the phospholipid layer may be deposited, again using techniques as described above.

Fig 4 illustrates a particular embodiment of microelectrode array not in accordance with the invention.

The illustrated electrode assembly 100 comprises a 10 x 10 array of working electrodes 101 produced in the manner described more fully above with reference to Fig 3 and thus comprising mercury coated iridium provided with a phospholipid layer on the mercury surface, the electrodes 101 being associated with a conducting trace 102.

The assembly further comprises a Ag/Ag/Cl reference electrode (not individually referenced but similar to that shown in Fig 2) associated with a conducting trace 103. A platinum electrode 104 associated with a conducting trace 105 is also provided.

Conducting traces 102, 103 and 105 are associated with respective gold contact pads 106, 107 and 108 respectively for connection to electronic control/measurement systems, e.g. as shown schematically in Fig 5.

Although the construction of the sensor has been described with specific reference to iridium as the carrier metal, it will be appreciated that the same principles of construction may be employed for platinum.

Reference is made to Fig 8 which is a schematic cross-sectional view to a much enlarged scale of a composite Pt/Hg electrode assembly 200 on which a phospholipid layer may be deposited.

Electrode assembly 200 comprises a silicon wafer 201 on which is deposited a layer 202 of silicon nitride (e.g. having a thickness of 500 nm). Formed in the silicon nitride is at least one well on the base of which is an adhesion layer (e.g. 30nm) of titanium 203 on which is deposited a 100nm thick layer of platinum 204. Filling the well is a layer of mercury 205 provided on its upper surface (as viewed in Fig 8) with a monolayer 206 of a phospholipid. Reference numeral 207 in Fig 8 represents electroyte solution in which the electrode would, in use, be immersed. Although not illustrated in Fig 8, platinum layer 204 is associated with a conductive trace for connection to a potentiostat.

Fig 9 illustrates an electrode assembly 300 constructed in accordance with the general principles shown in, and described with reference to, Fig 8 above.

The electrode assembly 300 is generally rectangular and is formed towards one end thereof with two working electrodes 301 and 302 each within a well of a silicon nitride layer 303. Towards the opposite end of electrode assembly 301 are two contact pads 304 and 305. Electrode 301 is connected by a conductive trace 306 to contact pad 304 whereas electrode 302 is connected by conductive trace 307 to contact pad 305.

Referring now to Figs 10(a)-(c) there are illustrated schematic views of one embodiment of flow cell 400 constructed for the purposes of proof-of principle" (see Example 7 below) and incorporating an electrode assembly 300 of the type described with reference to Fig 9.

The schematic drawings of 10(a)-(c) respectively illustrate side, end and plan views of the flow cell 400 which (particularly from Figs 10(a) and (b)) will be seen to comprise a base portion 401 and a top portion 402 both formed in the manner described more fully below.

Upper surface of base portion 401 is formed with a rebate such that electrode assembly 300 may sit therein so that its end provided with the contact pads 304 and 305 projects from the flow cell (see particularly Fig 10(b)). It should be understand from Fig 10(b) that, in the illustrated position of the electrode assembly 300, the electrodes 301 and 302 as well as the contact pads 304 and 305 are uppermost.

The under surface of upper portion 402 has a central recess which (with upper portion 401 and lower portion 402 assembled together in the manner illustrated in Fig 10(a)) forms a "measurement cell" 403 into which the electrodes 301 and 302 face. Leading to the left from measurement cell 403 (as viewed in Fig(a) is an electrolyte entry channel 404 and leading to the right is an electrolyte outlet channel 405. Communicating with electrolyte inlet channel 404 is an injection port 406 for a sample to be analysed whereas leading from electrolyte outlet channel 404 are bores 407 and 408, one for incorporating a Ag/AgCl reference electrode and the other a Pt auxiliary electrode (neither shown) separately from the electrode assembly 401.

An annular groove is formed in the under surface of upper portion 402 of flow cell 400 and receives a sealing O-ring 409.

Although not illustrated in the drawings, screw holes are provided for receiving screws to assemble upper and lower portions 41 and 42 together.

Each of the electrodes 301 and 302 may have a different phospholipid deposited therein.

For measurement purposes the working electrodes 301 and 302 as well as the auxiliary and reference electrodes are connected to a potentiostat in the manner illustrated in Fig 5. The arrangement will be such that electrodes 301 and 302 can be individually addressed.

In use of the flow cell, electrolyte is passed into inlet 404 and allowed to flow through "measurement well" 403 and then through outlet channel 405. Sample to be analysed may be injected into part 406. Individual measurements may then be made for the effect of the sample on different phospholipids on electrodes 301 and 302.

The invention is illustrated by the following non-limiting Examples.

<u>**Example 1**</u> (not in accordance with the invention)

To establish proof of principle, mercury was electrodeposited on Ir circular discs surrounded by glass as an insulator. The electrodes were washed with deionised water and a phospholipid was then deposited on the mercury layer by passing the mercury coated electrode through a film of a solution of dioleoyl phosphatidycholine (DOPC) in pentane at an electrolyte-gas interface. Subsequently the pentane was evaporated to leave the DOPC on the mercury surface.

The phospholipid layers were monitored by rapid cyclic voltammetry (RCV) at 80 V $s^{-1}$.

A comparative experiment was conducted using DOPC on a hanging drop mercury electrode (prior art).

The results are shown in Fig 6 (a) for which the lighter trace shows the results for the mercury coated iridium electrode whereas the darker trace is for the HMDE electrode. It will be seen from Fig 6 (a) that the two traces are virtually identical and both demonstrate the characteristic peaks (*1* and *2*).

Fig 6 (b) shows the results for the mercury coated iridium electrode scanned at 100 V $s^{-1}$. Once again the two characteristic peaks (*1* and 2) can clearly be seen.

The results show that the mercury coated electrode shows sharper voltammetric peaks at a rapid scan rate. This finding indicates that the occurrence of the phase transitions is a function of electrode size. Sharper voltammetric peaks are more suitable for analytical purposes and favour the microelectrode as a support for phospholipids. We have shown that removal of phospholipid from the microelectrode surface can be achieved by applying an extreme potential of -3.0 V vs Ag/AgCl. The voltammetric peaks displayed in the RCV s in Figs. 6 (a) and (b) can be used for the selective analytical recognition of a large number of dissolved organic species at very low (*nano* to $\mu$ mol $dm^{-3}$) level. These results show that stable ordered phospholipid layers can be deposited on to Hg/Ir microelectrodes.

<u>**Example 2**</u>

Mercury was electrodeposited on a close packed hexagonal array of 1800 platinum micro electrodes (on a base of Pt of 2mm diameter), the microelectrodes being of dimension 10 $\mu$m diameter with a 20 $\mu$m spacing centre-to-centre. Phospholipid DOPC was deposited on this array of microelectrodes by evaporating a solution of phospholipid on the surface of the array.

The array was introduced into an electrolyte solution where it was voltammetrically cycled between potentials of -0.2 and -2.0 V vs. Ag/AgCl. This annealed the DOPC monolayer to form a stable organised layer as demonstrated by Fig 7 which is a cyclic voltammetry plot at 30 V $s^{-1}$ from -0.2 V to -1.0 V. This plot displays the characteristic phase transitions. These layers have been shown to be stable for at least one hour.

## Example 3

Electrode assemblies of the type illustrated in Fig 9 was prepared using the procedure set out below to produce an assembly in which the wells in the silicon nitride layer 303 at a diameter of 960 $\mu$m.

A 100 mm thick silicon wafer substrate was cleaned with piranha solution (a 2:1 (v:v) mixture of sulphuric acid and hydrogen peroxide) and subjected to thermal oxidation to grow a layer of dense oxide on the wafer surface. Standard UV photolithography techniques were used to produce a plurality of identical resist patterns on the wafer each corresponding to one electrode assembly. The individual resist patterns had developed positive resist (absent resist) at the regions corresponding to the working electrodes 301 and 302, the contact pads 304 and 305 and the conductive traces 306 and 307.

Using conventional thermal evaporation techniques a 30 nm thick titanium adhesion layer and then a 100 nm thick platinum layer were applied to the substrate (see layers 203 and 204 in Fig 8).

The pattern was revealed using the standard practice of "metal lift-off" by dissolving the photo-resist in acetone.

A layer of silicon nitride approximately 500 nm thick was then deposited using Plasma Enhanced Chemical Vapour Deposition (PECVD) (see layer 202 in Fig 8).

Further UV photolithography was then used to pattern the electrode regions 301 and 302 and the contact pads 304 and 305 (resist developed selectively in these regions) using a second photo mask (etch mask). The underlying silicon nitride was then etched using a hydrofluoric acid based wet etch down to the surface of the platinum layer, so the latter was exposed at the base of wells in the silicon nitride and to provide the contact pads 304 and 305.

The remaining resist was then removed and the device cleaned with piranha solution.

Individual electrode assemblies were subsequently isolated from the others formed on the wafer by dicing the wafer using a wafer saw.

Electrodeposition of mercury onto platinum disc working electrodes was performed in a standard three electrode cell containing a double junction reference electrode (3.5 mol $dm^{-3}$ KCl, Ag/AgCl inner filling, 0.1 mol $dm^{-3}$ perchloric acid outer filling) and a platinum bar auxillary electrode both supplied by Metrohm. The working electrodes were introduced into the cell by means of a micromanipulator and connected via crocodile clips attached to platinum bond pads. The potential at the surface of the working electrodes was set using a PGSTAT12 (Ecochemie, Utrecht, The Netherlands)potentiostat controlled by AUTOLAB software. The silicon wafer based working electrodes were cleaned prior to electrodeposition in a hot solution of sulphuric acid (Fisher Scientific) and 30% hydrogen peroxide (Fluka) in a ratio of approximately 3:1 before drying under nitrogen. Electrodeposition was performed at - 0.4V vs. the 3.5 mol $dm^{-3}$ KCL Ag/AgCl reference and monitored by means of chronocoulometry. The deposition was terminated by opening the circuit and immediately removing of the electrode from the deposition solution once 1 Coulomb of charge had passed.

The liquid mercury deposited on the platinum was in the form of a "flattened hemisphere". The mercury was immobilised sufficiently on the platinum to allow the electrode assembly to be turned "upside down" without loss of mercury.

## Example 4

This Example demonstrates the stability of an electrode assembly produced in accordance with the procedure of Example 3.

The electrode assembly was tested in a three electrode cell which was temperature controlled at *25°C* and which contained 0.1 mol·$dm^{-3}$ KCl phosphate buffered at pH 7.4. The solution was deaerated prior to introduction of the electrode assembly by bubbling argon gas through the stirred solution. The electrode assembly was lowered into the cell using a micro manipulator and connected to the external potentiostat by crocodile clips attached to one of the electrode contact pads. The working electrode potential was set relative to a Ag/ AgCl 3.5 mol·$dm^{-3}$ KCl reference electrode separated from the cell by a porous glass frit. A platinum bar counter electrode completed the circuit. Rapid cyclic voltammetry measurements were carried out using an ACM research potentiostat (ACM instruments, Cumbria, UK) interfaced to a Powerlab 4/25 signal generator and ADC (AD Instruments, Oxfordshire, UK) controlled by Scope™ software.

The electrode assembly was washed with a jet of Mili Q water before introduction into the cell. It was then electrochemically cleaned for 30 seconds using the procedure described in the following paragraph.

For the cleaning procedure, the potential of the working electrode was scanned in a cathodic direction rapidly desorbing any contaminating organic material into the bulk solution using the following conditions:

- Range (-0.2 V to -2.625 V) @ 97V·s$^{-1}$

The following I/V curve was then recorded:

- Range (-0.2V to -2V) @ 40V·s-$^{1}$
  (5 single consecutive sweeps recorded + a sweep recorded after repetitive cycling once the trace is visibly stable - after ≈2 seconds)

Subsequently the above described cleaning procedure was operated continuously for 30 minutes.
The following I/V curve was then again recorded:

- Range (-0.2V to -2V) @ 40V·s-$^{1}$
  (5 single consecutive sweeps recorded + a sweep recorded after repetitive cycling once the trace is visibly stable - after ≈2 seconds)

The results are presented in Figs 11(a) and 11(b) which respectively show the curves obtained after the 30 seconds initial cleaning and after the 30 minutes cleaning.
For the purposes of comparison, Fig 12 shows an I/V curve obtained under the same conditions as those described above for a platinum electrode (more specifically an electrode assembly of the type produced in accordance with Example 3 above but without deposition of mercury). Fig 13 shows an I/V curve obtained using the conditions described above for a Hanging Mercury Drop Electrode normalised by surface area.
Referring firstly to Fig 13, it will be seen that the I/V curve for the HMDE displays a characteristic "water hump" (see left hand part of the curve illustrated in Fig 13). This "water hump" is not seen in the I/V curve for platinum illustrated in Fig 12.
Referring now to Figs 11(a) and 11(b), it will firstly be noted that both curves display the characteristic "water hump" displayed by mercury (cf Fig 13). Thus the electrode assembly produced in accordance with Example 3 displays the characteristics of a mercury electrode rather than a platinum electrode. Moreover a comparison of Figs 11(a) and 11(b) which are respectively before and after the 30 minute cleaning period demonstrates that the curves are identical indicating there is no significant loss in surface area and the surface character remains the same.
The surface area of the electrode can be calculated accurately in accordance with Equation (1) from the capacitance current using the value of specific capacitance for mercury measured for the water hump at ~ -0.3V as ~ 40$\mu$F cm$^{-2}$ [27]).

$$Area = \left( \frac{I}{\frac{\delta V}{\delta t} . C_{sp}} \right) \qquad (1)$$

For the Pt/Hg composite electrode used in the above experiments this yielded a value for surface area of the electrode as ~ 0.744mm$^2$. The surface area of the platinum disc electrode (prior to mercury deposition) was ~ 0.724 mm$^2$ measured using an optical microscope. The calculated value for the composite electrode (i.e. mercury deposited on the platinum) was slightly higher than that for the flat disc providing a reasonable result for a flattened hemisphere because it lies between the bounds of a perfectly flat film and a hemisphere. Values for surface area calculated in this fashion were used to produce the specific capacitance plots for composite electrodes coated with phospholipid monolayers in subsequent Examples.

**Example 5**

This Example demonstrates the ability of an electrode assembly produced in accordance with the procedure of Example 3 to support phospholipid monolayers.
The following 5 phospholipids were selected and varied only in the chemical functionality of their head group region:

(a) 1,2-Dioleoyl-sn-Glycero-3-Phosphocholine (DOPC)

(b) 1,2 Dioleoyl-sn-Glycero-3-Phosphoethanolamine (DOPE)

(c) 1,2-Dioleoyl-sn-Glycero-3-[Phospho-rac-(1-glycerol)] (Sodium Salt) (DOPG)

(d) 1,2-Dioleoyl-sn-Glycero-3-[Phospho-L-Serine] (Sodium Salt) (DOPS)

(e) 1,2-Dioleoyl-sn-Glycero-3-Phospho(Ethylene Glycol) (Sodium Salt) (DOPEG)

The chemical structural formulae of the phospholipids (a)-(e) are shown in Fig 12.

Solutions of each lipid of weight concentration 2 mg·ml$^{-1}$ dissolved in 1:4 chloroform:pentane were prepared separately yielding molar concentrations in the range 2.5 mmol·dm$^{-3}$ to 3 mmol·dm$^{-3}$. The electrochemical cell was set-up as described in Example 4, containing 0.1 mol·dm-$^{3}$ KCl phosphate buffered at a concentration of 0.01 mol·dm$^{-3}$ to pH 7.4 and deaerated with argon for 30 minutes prior to introduction of the working electrode. 12.5 $\mu$l of each lipid solution was added by syringe to the electrolyte and the working electrode was then lowered through the solution/argon interface. The electrode was cleaned for 30 seconds using the *in-situ* electrochemical cleaning method described in Example 4 before lifting the electrode through the solution interface briefly and re-submerging it to form an evenly coated layer.

No discernible differences were observed in repetitive scans at 40 V·s$^{-1}$ over the potential range -0.2 V to -1.2 V for the electrode coated at open circuit, with the potential held at a constant -0.2 V or while repetitively cycled over the above range (data not shown).

Cathodic scans of cyclic voltamograms recorded at 40 Vs$^{-1}$ were converted to specific capacitance plots for the phospholipids (a) DOPC, (b) DOPE, (c) DOPG, (d) DOPS and (e) DOPEG. The results are plotted in Figs 15(a)-(e) respectively for which the thick line is the specific capacitance plot and the thin line is for an electrochemically cleaned electrode assembly of the type produced in Example 3 (i.e. no deposited phospholipid).

It will be seen from the data presented in Fig 15 that the phospholipids act as variable dielectrics over the potential range between the potential of zero charge (p.z.c.) for mercury and the layer's desorption potential. The phospholipids impart selective chemical functionality to the surface and greatly affect the surface potential and capacitance profile producing unique finger print peaks relating to complex phase transitions of the absorbed layers.

## Example 6

This Example demonstrates the stability of the phospholipid monolayers.

Using the procedure of Example 5, the mercury surface of electrode assemblies produced in accordance with Example 4 were separately coated with the phospholipids DOPC, DOPS and DOPG.

For the DOPC coated electrode, a cyclic voltammogram was recorded at 40 V.s$^{-1}$ over the potential range -0.2 V to -1.2 V. The electrode was then electrochemically cleaned *in-situ* for 30 seconds using the cleaning technique described in Example 4 and the procedure repeated six times. The voltammograms were converted to specific capacitance plots and the average trace plotted. The results are shown in Fig 16(a) in which the narrow error bars (error bars = $\pm$ 1 SD) give a good indication as to the high level of measurement reproducibility between coatings. Thus the phospholipid monolayer deposition exhibited a high degree of similarity between experiments.

For the DOPS coated electrode, an initial scan was recorded at 40 V.s$^{-1}$ of the potential range -0.2V to -1.2V. The scan was repeated and recorded every 1 minute time interval for 30 consecutive minutes. The cyclic voltammograms were converted to specific capacitance plots and the average trace plotted. The results are shown in Fig 16(b) in which the narrow error bars ($\pm$ 1 SD) indicate that the capacitance minimum and current peak remain stable over the course of the experiment thus demonstrating the monolayer integrity similarly remains stable and reproducible.

To test stability and reproducibility in the case of continually cycling the potential between measurements, the DOPG coated electrode was continuously cycled at 40 V.s$^{-1}$ over the potential range -0.2 V to -1.2 V resulting in interrogation $\approx$15 times per second, the cycle lasting 50 ms for the ramp + 40 ms software forced delay. The scans were recorded initially and after 5, 15 and 25 minutes. The cyclic voltammograms were converted to specific capacitance plots and the traces overlaid to see any significant changes in the capacitance of the monolayer. The results are shown in Fig 16(c) from which it can be seen that the traces overlay almost exactly indicating that monolayer integrity is sufficiently stable over the time period measured.

## Example 7

This Example demonstrates use of a prototype flow cell 400 of the type illustrated in Fig 10 which was constructed to establish "proof-of-principle" for use of an electrode assembly in accordance with the invention in a flow cell. The flow cell had an overall length of 5cm, a height of 3 cm (each of lower and upper portions 401 and 402 having a height of 1.5cm) and a width of 2 cm. Injection port 406 as well as entry and exit channels 404 and 405 were of 4mm diameter. Bores 407 and 408 had a diameter of 2 mm and were angled at 45 degrees to exit channel 405. The electrode assembly 300 was produced in accordance with the procedure of Example 3 but passing 2C of charge (rather than 1 C). Electrode assembly 300 had a length of 1cm, a width of 5mm and a depth of 0.5mm.

A Ag/AgCl microelectrode was provided in bore 407 and platinum counter electrode in bore 408.

Contact pads 304 and 305 on the electrode assembly were individually connected to a potentionstat and could be addressed individually by means of a two way switch.

Lipid vesicle deposition dispersions of DOPC and DOPS were prepared by dissolving 25 mg of powdered pure phospholipid in 50:50 chloroform/methanol and rotary evaporating in a glass round bottomed flask at 25°C under light vacuum until dry. The residue was then re-suspended in 12.5 ml of phosphate buffered saline (0.1 $mol.dm^{-3}$ KCl, pH 7.4) to produce a $2mg.ml^{-1}$ dispersion which was then tip sonicated for ≈20 minutes to produce vesicles.

The lipid layers were deposited by injecting100 μL of 2 $mg.mL^{-1}$ DOPC or DOPS vesicle dispersions into the injection port 406 upstream of the electrodes while electrolyte (0.1m KCl phosphate buffered (10mM) to pH 7.4) composition, flow rate?) was passed into and along inlet channel 404, through measurement cell 403 and out through channel 405 at a rate of 5ml per minute.

It was found that the DOPC layers could be deposited using the same conditions as adopted for the cleaning procedure described in Example 4 but applied for *ca* 2 seconds. The potential cycling was stopped by opening the circuit when over-covered layer thinned sufficiently to exhibit the sharp phase transition peaks shown in Fig 17 which is rapid cyclic voltammogram at 36 V $s^{-1}$ of the electrode coated with DOPC (thick line) measured with the experimental flow cell. For the purposes of comparison, Fig 17 also incorporates the corresponding trace for the Pt/Hg electrode (thin line) in the absence of lipid.

The DOPS layers were deposited under different potential conditions from DOPC due to the DOPS layers spreading rapidly at potentials < -1.4 V. Initial trials suggest that DOPS can be deposited over a lower potential range sweep with a cathodic apex of -1.1 V. The DOPS layers were allowed slowly to build with time and successive additions to produce an I/V curve (thick line) as seen in Fig 18 which is a rapid cyclic voltammogram (thick line) at 38 V $s^{-1}$ of the electrode coated with DOPS measured within the experimental flow cell. For the purposes of comparison, Fig 18 also incorporates the trace (thin line) for the rapid cyclic voltammogram at 36 V $s^{-1}$ of the Pt/Hg electrode without lipid.

Both lipid layers (DOPC and DOPS) on the electrodes proved to be stable over a period of > 10 minutes with electrolyte flowing at ~ 4.5 $mL{\cdot}min^{-1}$ and each could be deposited with a reasonable degree of reproducibility after cleaning the electrode *in-situ* and repeating the procedure.

A feature of the prototype flow cell was the instability of the micro-reference electrode potential which was measured as +260mV when compared with the Ag/AgCl (3.5 $mol.dm^{-3}$) reference electrode used in the static cell. Thus all potentials quoted from data within the flow cell are *vs.* a drifting Ag/AgCl reference. The exact drift can be evaluated by comparing the positions of the first or second phase transition peaks of DOPC which occur at defined potentials on the Hg surface.

From Figs 17 and 18 a clear slant to the traces can be observed as well as broadening of the current peaks. This can be attributed to three contributing factors. Firstly the cell was found not to be water tight due to an imperfect seal with the O-ring. (Any small electrolyte leaks can produce interfering faradaic currents when it comes into contact with the crocodile clips and contact pads.) Secondly, the electrolyte contains a larger quantity of dissolved oxygen than the static cell which is more efficient at de-aeration. Thirdly, the distance of the reference electrode from the working electrodes is slightly greater and there is a higher solution resistance in the flow cell due to the smaller reference electrode fritt that may become more easily blocked by phospholipid flows, the greater solution resistance influences the potential applied to the working electrodes through the phenomena of Ohmic drop.

In spite of the "deficiencies" of the prototype flow cell, this Example clearly demonstrates a number of significant points. Firstly, the mercury layers are stable in the electrolyte flow. Secondly the phospholipids can be deposited on to the mercury layers and (when interrogated by cyclic voltammetnry) give peaks corresponding with those obtained in a static cell, allowing for the "deficiencies" of the prototype flow cell. Thirdly the lipid layers are stable in the electrolyte flow. Fourthly the lipid layers can conveniently be deposited from the electrolyte flow by applying the appropriate potential to the electrode assemblies 301 and 302 of the electrode assembly 300.

## Example 8

This Example demonstrates similarity in properties of a Hanging Mercury Drop Electrode (HMDE) and Pt/Hg electrode as produced by the procedure of Example 3.

The HMDE was based on using a capillary with a diameter of 0.1mm so as to provide a surface area for the mercury drop about the same as the surface area of the mercury in the electrode in accordance with the invention.

The HMDE and the electrode of the invention were compared side-by-side in a static cell configuration. All experiments were carried out in 0.1 M potassium chloride solution. Measurements were taken at 75 Hz between -0.4 to -1.15 V with 4.95 mV rms at a scan rate of 5 mV $s^{-1}$.

Capacitance - potential scans for the HMDE and the composite Pt/Hg electrode are shown in Figs 19(a) and (b) respectively. Although there is a slight difference in the capacitance values shown in Figs 19(a) and (b), the shape of the plots indicates close similarity in the properties of the two electrodes.

**Example 9**

This Example compares a HMDE electrode with an electrode as produced in accordance with Example 3, both coated with a monolayer film of 1,2-dioleoyl-sn-glycero-3-phosphocholine (DOPC), new layers of which were deposited between consecutive scans. Experiments were performed in 0.1M Kcl. A negative going 'forward' potential scan was performed between -0.4 V and -1.15 V, and then followed immediately by a positive going 'reverse' scan from -1.15 V to -0.4 V with 4.95 mV rms at a scan rate of $5mVs^{-1}$. The results for the HMDE and the electrode of the invention are shown in Figs 20(a) and (b) respectively.

As with the scans of the "bare" electrodes in the electrolyte solution (results shown in Fig 19), both electrodes show comparable results. The 'reverse' potential scans show that the electrode of the invention is slightly more stable than the HMDE electrode.

**Example 10**

The procedure of Example 9 was repeated but with the incorporation in the electrolyte of chlorpromazine at a concentration of 0.5 $\mu$mol $dm^{-3}$. The structure of chlorpromazine is as shown below:

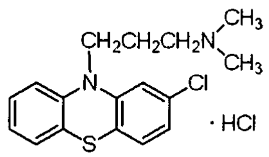

The results for HMDE and the electrode of the invention are represented by the dark lines in Figs 21(a) and (b) respectively. For the purposes of comparison, these Figures also incorporate the results obtained in Example 9.

Once again, both electrodes show very similar capacitance - potential scans.

**Example 11**

The procedure of Example 9 was repeated but with the incorporation in the electrolyte of promethazine at a concentration of 0.5 $\mu$mol $dm^{-3}$. The structure of promethazine is shown below:

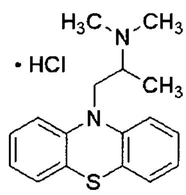

The results for HMDE and the electrode of the invention are represented by the dark lines in Figs 22(a) and 22(b) respectively. For the purposes of comparison, these Figures also incorporate the results obtained in Example 9.

In this case, both electrodes show response to the test compound but with the electrode of the invention displaying the stronger response (greater depression of the peaks) than the HMDE.

**Example 12**

The procedure of Example 9 was repeated but with the incorporation in the electrolyte of H16 at a concentration of 0.5 $\mu$mol $dm^{-3.}$ The structure of H16 is shown below.

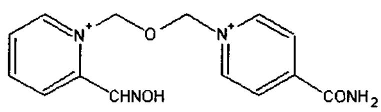

The results for HMDE and the electrode of the invention are represented by the dark lines in Figs 23(a) and 23(b) respectively. For the purposes of comparison, these Figures also incorporate the results obtained in Example 9.

In this case, the electrode of the invention shows a far greater response than that seen with the HMDE, thus

indicating greater sensitivity of the former than the latter.

**Example 13**

Example 12 was repeated but using 5 μmol $dm^{-3}$ of H16.

The results for HMDE and the electrode of the invention are represented by the dark lines in Figs 24(a) and (b) respectively. For the purposes of comparison, these Figures also incorporate the results obtained in Example 9.

Figs 24(a) and (b) demonstrate a large response to the test compound that is easily visible on both electrodes, with the electrode of the invention still showing the slightly larger response.

**Example 14**

Following the procedure of Example 9, Pt/Hg composite electrodes produced in accordance with the procedure of Example 3 and provided individually with monolayers of DOPC, DOPE and DOPS were evaluated.

The results are shown in Figs 25(a)-(c) which show the results for DOPC, DOPE and DOPS respectively (note the difference in vertical scale for DOPS compared to the other two lipids shown).

The results show that monolayers of all three types of phospholipid can be formed on the electrodes and that each produces a different characteristic trace in the AC Voltammetry experiments.

**Example 15**

The procedure of Example 14 was repeated but incorporating 0.5 μmol $dm^{-3}$ chlorpromazine in the electrolyte solution.

The results were illustrated by the dark lines in Figs 26(a)-(c) which show the results for DOPC, DOPE and DOPS respectively. For the purposes of comparison, Figs 26(a)-(c) also incorporate the results shown in Fig 25.

Fig 26 clearly demonstrates interaction of the chlorpromazine with the lipid monolayers (cf the superimposed results from Fig 25 for the "non-interacting" monolayers).

**Example 16**

The procedure of Example 10 was repeated for electrodes produced in accordance with Example 3 and incorporating a DOPC monolayer to evaluate the system for the following compounds all provided in the electrolyte at a concentration of 0.5 μmol $dm^{-3}$, save for (f) limonene which was used at a concentration of 5 μmol $dm^{-3}$:

(a) Chlorpromazine
(b) Promethazine
(c) HI6
(d) MB327
(e) Fluoranthene
(f) Limonene

The results are represented by the dark lines shown in Figs 27(a)-(f) respectively which also incorporate the structural formulae of the compounds tested and the results obtained for the DOPC coated electrode without test compound in the electrolyte.

The results show that all six of the compounds are detectable by the system. Also, that it is possible to differentiate between them at comparable concentrations.

**Example 17**

The procedure of Example 10 was repeated using chlorpromazine concentrations of:

(a) 0.05 μmol $dm^{-3}$
(b) 0.1 μmol $dm^{-3}$
(c) 0.2 μmol $dm^{-3}$
(d) 0.5 μmol $dm^{-3}$

The results are represented by the dark lines shown in Figs 28(a)-(d) respectively which also incorporate results obtained for the DOPC electrode without chlorpromazine incorporated in the electrolyte.

As can be seen from Figs 28(a)-(d) there was a response from the system, even at the lowest chlorpromazine concentration tested, i.e. 0.05 $\mu$mol dm$^{-3}$.

Using the formula for ppb of:

$$\text{Parts per billion} = \left(\frac{\text{mass of component}}{\text{mass of solution}}\right) \times \left(1 \times 10^{9}\right)$$

and assuming a density for the solution used as 1g per ml, the sensitivity of 0.05 $\mu$mol dm$^{-3}$ converts to approximately 18 ppb.

## References

**[1].** Sackmann E; Science 1996, 271, 43.
**[2].** Wanunu, M.; Vaskevich, A;Rubinstein,I.; J. Am. Chem. Soc. 2004, 126, 5569.
**[3].** Heyse, S.; Vogel, H.; Sanger, M.; Sigrist, H.; Protein Sci. 1995,4,2532.
**[4].** Shumyantseva, V.V.; Ivanov,Y.D.; Bistolas ,N.; Scheller, F.W.; Archakov, A.I.; Wollenberger, U.; Anal.Chem. 2004, 76,6046.
**[5].** Becucci, L.; Leon, R.R.; Moncelli, MR,; Rovero, P.; Guidelli, R.;Langmuir 2006, 22, 6644.
**[6].** Nelson.A.; Anal. Chim. Acta 1987, 194, 139.
**[7].** Nelson, A.; Auffret,N.; Readman,J.; Anal. Chim. Acta 1988, 207, 45.
**[8].** Nelson,A.; Auffret,N.; Borlakoglu,J.; Biochim. Biophys. Acta 1990, 1021, 205.
**[9].** Bizzotto, D; Nelson, A; Langmuir 1998, 14, 6269.
**[10].** Monne J; Galceran J; Puy J; Nelson A; Langmuir 2003,19,4694.
**[11].** Whitehouse, C; O'Flanagan, R; Lindholm-Sethson, B; Movaghar, B; Nelson, A; Langmuir 2004, 20,136.
**[12].** Whitehouse, C; Gidalevitz, D; Cahuzac, M; Koeppe, R.E II; Nelson, A; Langmuir 2004, 20, 9291
**[13].** Nelson, A; Biophys. J. 2001, 80, 2694.
**[14].** Protopapa,E.P.; Aggeli,A.; Boden,N.; Knowles,P.F.;Salay,L.C.; Nelson,A,; Medical Engineering and Physics 2006,28,944.
**[15].** Merrifield,J.; Tattersall,J.E.H.; Bird,M.; Nelson,A.; Electroanalysis in press
**[16].** Weiss,S; Millner,P.; Nelson,A.; Electrochimica Acta 2005, 50,4248.
**[17].** Burgers,I; Li,M.; Horswell,S.L.; Szymanski,G.;Lipkowski,J.; Satija,S.;Majewski, J.; Colloids and Surfaces B-Biointerfaces 2005,40,117.
**[18].** Bin,X.M.; Zawisza,I.; Goddard, J.D.;.lipkowski,J.; Langmuir 2005, 21,330.
**[19].** Nelson, A.; J.Electroanal.Chem. 2006 in press.
**[20].** Willmann, S. et al. J. Med. Chem. 2004, 47, 4022.
**[21].** F. Neville, M. Cahuzac, A. Nelson, D. Gidalevitz, 2004, Journal of Physics-Condensed Matter 16 (26): S2413-S2420.
**[22].** F. Neville, D. Gidalevitz, G. Kale, A. Nelson, 2006, Bioelectrochemistry. in press.
**[23].** Ringstad,L.; Nelson, A.; Malmsten,M.; in preparation.
**[24].** Stoodley,R.; Bizzotto,D.; Analyst 2003, 128, 552.
**[25].** Kounaves, S.P.; Deng, W.; Anal. Chem. 1993, 65, 375.
**[26].** Nolan, M.A.; Kounaves, S.P.; Anal. Chem. 1999, 71, 3567.
**[27].** D. C. Grahame, Journal Of The American Chemical Society, 1949, 71(9), 2975-2978.


## Claims

1. An electrode assembly (1) comprising at least one working electrode (2) comprised of a conductive carrier substrate (3) having a surface coated with mercury (4) immobilised on the surface of the substrate (3) and a phospholipide layer (5) coated on the surface of the mercury (4) remote from said substrate (3), **characterised in that** the conductive carrier substrate (3) is platinum.

2. An electrode assembly (1) as claimed in claim 1 comprising a plurality of the working electrodes (2).

3. An electrode assembly (1) as claimed in claim 1 or 2 wherein there is no exposed free platinum conductive carrier

substrate surface for the or each working electrode (2).

**4.** An electrode assembly (1) as claimed in any one of claims 1 to 3 wherein the or each working electrode (2) is a microelectrode.

**5.** An electrode assembly (1) as claimed in claim 4 wherein the mercury (4) has a maximum surface dimension of 2 μm to 1000 μm in any direction.

**6.** An electrode assembly (1) as claimed in claim 5 wherein the or each working electrode (2) is circular with a diameter of 2 μm to 1000 μm.

**7.** An electrode assembly (l) as claimed in any one of claims 1 to 6 wherein the phospholipid (5) is selected from the group consisting or dioleoyl phosphatidylcholine (DOPC), dioleoyl phosphatidylethanolamine (DOPE), 1,2-dioleoyl-sn-glycero-3-[phospho-rac-(1-glycerol)] (DOPG), 1,2-dioleoyl-sn-glycero-3-[phospho-1-serine] (DOPS) and 1,2-dioleoyl-sn-glycero-3-phospho(ethylene glycol) (DOPEG).

**8.** An electrode assembly (1) as claimed in any one of claims 1 to 7 comprising a layer of said platinum conductive carrier substrate (3) sandwiched the c between first (6) and second insulating substrate layers, the first one of which (6) is penetrated by at least one through aperture defining a well (12) for which said platinum conductive carrier substrate (3) provides a basal surface, the well (12) incorporating a mercury coating for said carrier substrate on which the phospholipid. layer is provided, thereby forming a said working electrode.

**9.** An electrode assembly (1) as claimed in claim 8 further comprising a conducting layer (8) sandwiched between the second insulting substrate and said platinum conductive carrier substrate layer with which the conducting layer is in electrically conducting relationship.

**10.** An electrode assembly as claimed in any one of claims 1 to 9 further comprising a reference electrode and/or a counter electrode.

**11.** An electrode assembly as claimed in claim 8 or 9, wherein the electrode assembly is in the form of a microelectrode array (100) and comprises:

(i) a counter electrode provided on the first insulating substrate layer, and
(ii) a reference electrode provided on the first insulating substrate layer.

**12.** A biosensor comprising:

(i) an electrode assembly as claimed in any one of claims 1 to 10,
(ii) at least one counter electrode for the working electrode(s),
(iii) a reference electrode,
(iv) means for applying a periodically varying voltage to the at least once working electrode, and
(v) means for determining variations in the differential capacitance of the phospolipid as a function of potential against the counter electrode.

**13.** A biosensor as claimed in claim 12 wherein the means for applying a periodically varying voltage is adapted to provide a sawtooth waveform.

**14.** A biosensor as claimed in claim 13 wherein the sawtooth waveform has a ramp rate of ≥ 1 V $s^{-1}$ for effecting measurements by cyclic voltammetry.

**15.** A biosensor as claimed in any one of claims 12 to 14 wherein the reference electrode and the working electrode are incorporated in the electrode assembly.

**16.** A biosensor as claimed in claim 12 wherein the electrode assembly is as defined in claim 8 or 9, the electrode assembly is in the form of a microelectrode array, the counter electrode is provided on the first insulating substrate layer, and the reference electrode is provided on the first insulating substrate layer.

**17.** A method of analysing a sample to determine biomembrane activity therein using a biosensor as claimed in any

one of claims 12 to 16, the method comprising the steps of:

(a) exposing the sample to the working electrode(s) of the electrode assembly; and
(b) using a voltammetric technique to determine the biomembrane activity.

**18.** A method as claimed in claim 17 wherein the voltammetry technique is rapid cyclic voltammetry.

**19.** A method as claimed in claim 17 wherein the ramp rate is $\geq 1\ V\ s^{-1}$.

**20.** A method as claimed in any one of claims 17 to 19 which comprise at least one repeat of the following sequence:

(c) preparing the working electrode by depositing a phospholipid on the mercury coating of a composite electrode comprised of the platinum conductive carrier substrate and mercury coating therefor;
(d) exposing the sample to the working electrode(s) of the electrode assembly;
(e) using a voltammetric technique to determine the biomembrane activity; and
(f) removing the phospholipid from the working electrode to leave a said composite electrode.

**21.** A method as claimed in claim 20 wherein step (c) is effected by scanning the composite electrode in the cathodic direction.

**22.** A method as claimed in claims 20 or 21 wherein (f) is effected by scanning the working electrode in the cathodic direction.

**23.** A method as claimed in any one of claims 17 to 22 effected in a flow cell.

**24.** A flow cell comprising:

(i) a measurement cell;
(ii) an electrode assembly as claimed in any one of claims 1 to 10 having at least one working electrode exposed within the measurement cell;
(iii) an electrolyte inlet to said measurement cell;
(iv) an electrolyte outlet from said measurement cell;
(v) a reference electrode; and
(vi) a counter electrode.

**25.** A flow cell as claimed in claim 24 wherein said working electrode, reference electrode and counter electrode are provided on a chip

**26.** A flow cell as claimed in claim 25 wherein said chip is provided by an electrode assembly as defined in claim 8 and in the form of a microelectrode array.

**Patentansprüche**

**1.** Elektrodeneinheit (1) umfassend mindestens eine Arbeitselektrode (2), bestehend aus einem leitfähigen Trägersubstrat (3) mit einer beschichteten Oberfläche mit Quecksilber (4) immobilisiert auf der Oberfläche des Substrats (3) und einer Phospholipid-Schicht (5), beschichtet auf der Oberfläche des Quecksilbers (4) entfernt vom Substrat (3), **dadurch gekennzeichnet, dass** das leitfähige Trägersubstrat (3) Platin ist.

**2.** Elektrodeneinheit (1) nach Anspruch 1 umfassend eine Vielzahl von den Arbeitselektroden (2).

**3.** Elektrodeneinheit (1) nach Anspruch 1 oder 2, wobei es keine ausgesetzte freie leitfähige Trägersubstrat-Platinoberfläche für die oder jede Arbeitselektrode (2) gibt.

**4.** Elektrodeneinheit (1) nach einem der Ansprüche 1 bis 3, wobei die oder jede Arbeitselektrode (2) eine Mikroelektrode ist.

**5.** Elektrodeneinheit (1) nach Anspruch 4, wobei das Quecksilber (4) ein maximales Oberflächenmaß von 2 μm bis

1000 μm in jede Richtung hat.

**6.** Elektrodeneinheit (1) nach Anspruch 5, wobei die oder jede Arbeitselektrode (2) kreisförmig mit einem Durchmesser von 2 μm bis 1000 μm ist.

**7.** Elektrodeneinheit (I) nach einem der Ansprüche 1 bis 6, wobei das Phospholipid (5) ausgewählt ist von der Gruppe bestehend aus Dioleoylphosphatidylcholin (DOPC), Dioleoylphosphatidylethanolamin (DOPE), 1,2-Dioleoyl-sn-glycero-3-[phospho-rac-(1-glycerol)] (DOPG), 1,2-Dioleoyl-sn-glycero-3-[phospho-1-serin] (DOPS) und 1,2-Dioleoyl-sn-glycero-3-phospho(ethylenglykol) (DOPEG).

**8.** Elektrodeneinheit (1) nach einem der Ansprüche 1 bis 7, umfassend eine Schicht des leitfähigen Platinträgersubstrats (3) eingelegt zwischen einer ersten (6) und einer zweiten isolierenden Substratschicht, wovon die erste (6) von mindestens einer Durchgangsöffnung durchdrungen wird, die einen Schacht (12) definiert, für den das leitfähige Platinträgersubstrat (3) eine Grundfläche bereitstellt, wobei der Schacht (12) eine Quecksilberbeschichtung für das Trägersubstrat integriert, auf dem die Phospholipid-Schicht bereitgestellt ist, wodurch die Arbeitselektrode gebildet wird.

**9.** Elektrodeneinheit (1) nach Anspruch 8, ferner umfassend eine leitende Schicht (8), eingelegt zwischen dem zweiten isolierenden Substrat und der leitfähigen Platinträgersubstrat-Schicht, mit der die leitende Schicht in elektrisch leitender Beziehung ist.

**10.** Elektrodeneinheit nach einem der Ansprüche 1 bis 9, ferner umfassend eine Referenzelektrode und/oder eine Gegenelektrode.

**11.** Elektrodeneinheit nach Anspruch 8 oder 9, wobei die Elektrodeneinheit in der Form einer Mikroelektrodenanordnung (100) ist und umfasst:

(i) eine Gegenelektrode, bereitgestellt an der ersten isolierenden Substratschicht, und
(ii) eine Referenzelektrode, bereitgestellt an der ersten isolierenden Substratschicht.

**12.** Biosensor umfassend:

(i) eine Elektrodeneinheit nach einem der Ansprüche 1 bis 10,
(ii) mindestens eine Gegenelektrode für die Arbeitselektrode(n),
(iii) eine Referenzelektrode,
(iv) Mittel zum Anwenden einer periodisch variierenden Spannung auf die mindestens eine Arbeitselektrode, und
(v) Mittel zum Bestimmen von Variationen in der Differenzkapazität des Phospholipids in Abhängigkeit vom Potenzial gegen die Gegenelektrode.

**13.** Biosensor nach Anspruch 12, wobei das Mittel zum Anwenden einer periodisch variierenden Spannung angepasst ist, um eine Sägezahnwellenform bereitzustellen.

**14.** Biosensor nach Anspruch 13, wobei die Sägezahnwellenform eine Rampenrate von $\geq 1\ V\ s^{-1}$ zum Durchführen von Messungen durch zyklische Voltametrie hat.

**15.** Biosensor nach einem der Ansprüche 12 bis 14, wobei die Referenzelektrode und die Arbeitselektrode in die Elektrodeneinheit eingegliedert sind.

**16.** Biosensor nach Anspruch 12, wobei die Elektrodeneinheit gemäß der Definition in Anspruch 8 oder 9 ist, die Elektrodeneinheit in der Form einer Mikroelektrodenanordnung ist, die Gegenelektrode an der ersten isolierenden Substratschicht bereitgestellt ist, und die Referenzelektrode an der ersten isolierenden Substratschicht bereitgestellt ist.

**17.** Verfahren zum Analysieren einer Probe, um die Biomembranaktivität darin unter Verwendung eines Biosensors nach einem der Ansprüche 12 bis 16 zu bestimmen, das Verfahren umfassend die folgenden Schritte:

(a) Aussetzen der Probe an die Arbeitselektrode(n) der Elektrodeneinheit; und
(b) Verwenden einer voltametrischen Technik zum Bestimmen der Biomembranaktivität.

**18.** Verfahren nach Anspruch 17, wobei die Voltametrietechnik eine schnelle zyklische Voltametrie ist.

**19.** Verfahren, nach Anspruch 17, wobei die Rampenrate $\geq 1$ V $s^{-1}$ ist.

**20.** Verfahren nach einem der Ansprüche 17 bis 19 das mindestens eine Wiederholung des folgenden Ablaufs umfasst:

(c) Vorbereiten der Arbeitselektrode durch Aufbringen eines Phospholipids auf die Quecksilberbeschichtung einer Verbundelektrode, die aus dem leitfähigen Platinträgersubstrat und der Quecksilberbeschichtung dafür besteht;
(d) Aussetzen der Probe an die Arbeitselektrode(n) der Elektrodeneinheit;
(e) Verwenden einer voltametrischen Technik zum Bestimmen der Biomembranaktivität; und
(f) Entfernen des Phospholipids von der Arbeitselektrode, um die Verbundelektrode zu hinterlassen.

**21.** Verfahren nach Anspruch 20, wobei Schritt (c) durch Scannen der Verbundelektrode in der kathodischen Richtung erfolgt.

**22.** Verfahren nach Anspruch 20 oder 21, wobei (f) durch Scannen der Arbeitselektrode in der kathodischen Richtung erfolgt.

**23.** Verfahren nach einem der Ansprüche 17 bis 22, ausgeführt in einer Durchflusszelle.

**24.** Durchflusszelle, umfassend:

(i) eine Messzelle;
(ii) eine Elektrodeneinheit nach einem der Ansprüche 1 bis 10 mit mindestens einer Arbeitselektrode, die in der Messzelle ausgesetzt ist;
(iii) einen Elektrolyteinlass zu der Messzelle;
(iv) einen Elektrolytauslass von der Messzelle;
(v) eine Referenzelektrode; und
(vi) eine Gegenelektrode.

**25.** Durchflusszelle nach Anspruch 24, wobei die Arbeitselektrode, Referenzelektrode und Gegenelektrode auf einem Chip bereitgestellt sind.

**26.** Durchflusszelle nach Anspruch 25, wobei der Chip durch eine Elektrodeneinheit gemäß Definition in Anspruch 8 und in der Form einer Mikroelektrodenanordnung bereitgestellt ist.

## Revendications

**1.** Ensemble d'électrodes (1) comprenant au moins une électrode de travail (2) composée d'un substrat de support conducteur (3) comportant une surface revêtue de mercure (4) immobilisé sur la surface du substrat (3) et une couche de phospholipide (5) déposée sur la surface du mercure (4) à distance dudit substrat (3), **caractérisé en ce que** le substrat de support conducteur (3) consiste en du platine.

**2.** Ensemble d'électrodes (1) selon la revendication 1 comprenant une pluralité des électrodes de travail (2).

**3.** Ensemble d'électrodes (1) selon la revendication 1 ou 2, dans lequel il n'y a aucune surface de substrat de support conducteur en platine libre exposée pour l'électrode de travail (2) ou pour chaque électrode de travail.

**4.** Ensemble d'électrodes (1) selon l'une quelconque des revendications 1 à 3, dans lequel l'électrode de travail ou chaque électrode de travail (2) est une microélectrode.

**5.** Ensemble d'électrodes (1) selon la revendication 4, dans lequel le mercure (4) a une dimension de surface maximum de 2 $\mu$m à 1000 $\mu$m dans toutes les directions.

**6.** Ensemble d'électrodes (1) selon la revendication 5, dans lequel l'électrode de travail ou chaque électrode de travail (2) est circulaire avec un diamètre de 2 $\mu$m à 1000 $\mu$m.

7. Ensemble d'électrodes (1) selon l'une quelconque des revendications 1 à 6, dans lequel le phospholipide (5) est sélectionné dans le groupe consistant en le dioleoylphosphatidylcholine (DOPC), le dioleoylphosphatidylethanolamine (DOPE), le 1,2-dioleoyl-sn-glycero-3-[phospho-rac-(1-glycerol)] (DOPG), le 1,2-dioleoyl-sn-glycero-3-[phospho-1-serine] (DOPS) et le 1,2-dioleoyl-sn-glycero-3-phospho(éthylène glycol) (DOPEO).

8. Ensemble d'électrodes (1) selon l'une quelconque des revendications 1 à 7 comprenant une couche dudit substrat de support conducteur en platine (3) intercalée entre des première (6) et deuxième couches de substrat isolantes, dont la première (6) est pénétrée par au moins une ouverture traversante définissant un puits (12) pour lequel ledit substrat de support conducteur en platine (3) réalise une surface de base, le puits (12) incorporant un revêtement de mercure pour ledit substrat de support sur lequel la couche de phospholipide est prévue, formant de ce fait une dite électrode de travail.

9. Ensemble d'électrodes (1) selon la revendication 8 comprenant en outre une couche conductrice (8) intercalée entre le deuxième substrat isolant et ladite couche de substrat de support conducteur en platine avec laquelle la couche conductrice est dans une relation de conduction électrique.

10. Ensemble d'électrodes selon l'une quelconque des revendications 1 à 9 comprenant en outre une électrode de référence et/ou une contre-électrode.

11. Ensemble d'électrodes selon la revendication 8 ou 9, dans lequel l'ensemble d'électrodes est sous la forme d'un réseau de microélectrodes (100) et comprend :

(i) une contre-électrode prévue sur la première couche de substrat isolante, et
(ii) une électrode de référence prévue sur la première couche de substrat isolante.

12. Biocapteur comprenant :

(i) un ensemble d'électrodes selon l'une quelconque des revendications 1 à 10,
(ii) au moins une contre-électrode pour la ou les électrodes de travail,
(iii) une électrode de référence,
(iv) un moyen pour appliquer une tension variant périodiquement à ladite au moins une électrode de travail, et
(v) un moyen pour déterminer les variations de la capacitance différentielle du phospholipide en fonction du potentiel par rapport à la contre-électrode.

13. Biocapteur selon la revendication 12, dans lequel le moyen pour appliquer une tension variant périodiquement est conçu pour fournir une forme d'onde en dents de scie.

14. Biocapteur selon la revendication 13, dans lequel la forme d'onde en dents de scie a une vitesse de rampe supérieure ou égale à 1 $Vs^{-1}$ pour effectuer des mesures par voltampérométrie cyclique.

15. Biocapteur selon l'une quelconque des revendications 12 à 14, dans lequel l'électrode de référence et l'électrode de travail sont incorporées dans l'ensemble d'électrodes.

16. Biocapteur selon la revendication 12, dans lequel l'ensemble d'électrodes est selon les revendications 8 ou 9, l'ensemble d'électrodes est sous la forme d'un réseau de microélectrodes, la contre-électrode est prévue sur la première couche de substrat isolante, et l'électrode de référence est prévue sur la première couche de substrat isolante.

17. Procédé d'analyse d'un échantillon pour déterminer une activité de biomembrane dans celui-ci en utilisant un biocapteur selon l'une quelconque des revendications 12 à 16, le procédé comprenant les étapes :

(a) d'exposition de l'échantillon à la ou aux électrodes de travail de l'ensemble d'électrodes ; et
(b) d'utilisation d'une technique voltampérométrique pour déterminer l'activité de biomembrane.

18. Procédé selon la revendication 17, dans lequel la technique de voltampérométrie consiste en une voltampérométrie cyclique rapide.

19. Procédé selon la revendication 17, dans lequel la vitesse de rampe est supérieure ou égale à 1 $Vs^{-1}$.

**20.** Procédé selon l'une quelconque des revendications 17 à 19 qui comprend au moins une répétition de la séquence suivante :

(c) la préparation de l'électrode de travail en déposant un phospholipide sur le revêtement de mercure d'une électrode composite composée du substrat de support conducteur en platine et d'un revêtement de mercure pour celui-ci ;
(d) l'exposition de l'échantillon à la ou aux électrodes de travail de l'ensemble d'électrodes ;
(e) l'utilisation d'une technique voltampérométrique pour déterminer l'activité de biomembrane ; et
(f) le retrait du phospholipide de l'électrode de travail pour laisser une dite électrode composite.

**21.** Procédé selon la revendication 20, dans lequel l'étape (c) est effectuée en balayant l'électrode composite dans la direction cathodique.

**22.** Procédé selon les revendications 20 ou 21, dans lequel l'étape (f) est effectuée en balayant l'électrode de travail dans la direction cathodique.

**23.** Procédé selon l'une quelconque des revendications 17 à 22 effectué dans une cellule d'écoulement.

**24.** Cellule d'écoulement comprenant :

(i) une cellule de mesure ;
(ii) un ensemble d'électrodes selon l'une quelconque des revendications 1 à 10 comportant au moins une électrode de travail exposée dans la cellule de mesure ;
(iii) une entrée d'électrolyte vers ladite cellule de mesure ;
(iv) une sortie d'électrolyte de ladite cellule de mesure ;
(v) une électrode de référence ; et
(vi) une contre-électrode.

**25.** Cellule d'écoulement selon la revendication 24, dans laquelle ladite électrode de travail, ladite électrode de référence et ladite contre-électrode sont prévues sur une puce.

**26.** Cellule d'écoulement selon la revendication 25, dans laquelle ladite puce est réalisée par un ensemble d'électrodes selon la revendication 8 et sous la forme d'un réseau de microélectrodes.

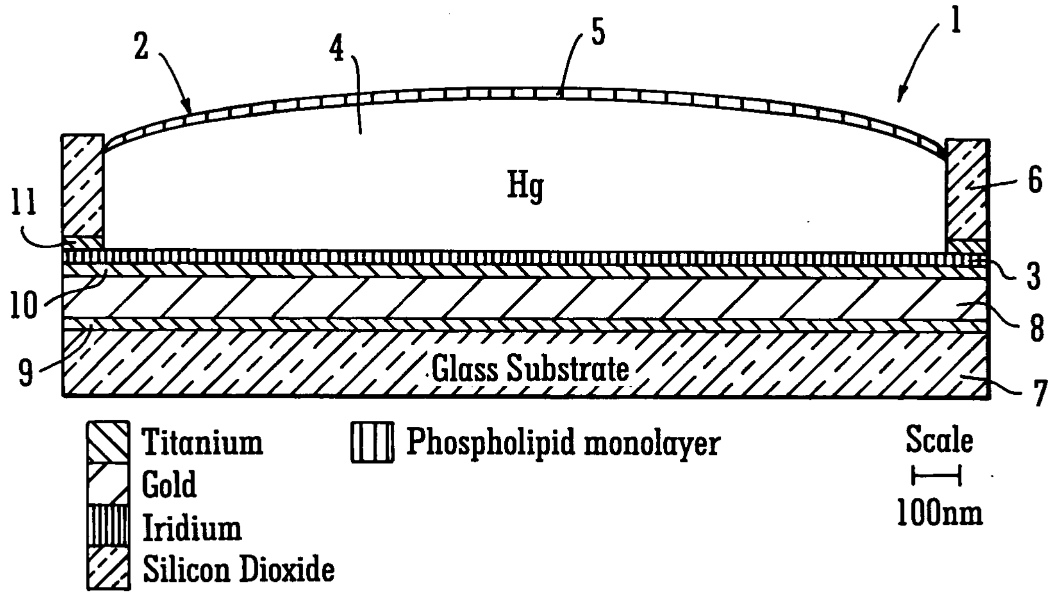
1
2
4
5
Hg
6
11
3
10
8
9
Glass Substrate
7
Titanium
Gold
Iridium
Silicon Dioxide
Phospholipid monolayer
Scale
100nm

FIG. 1

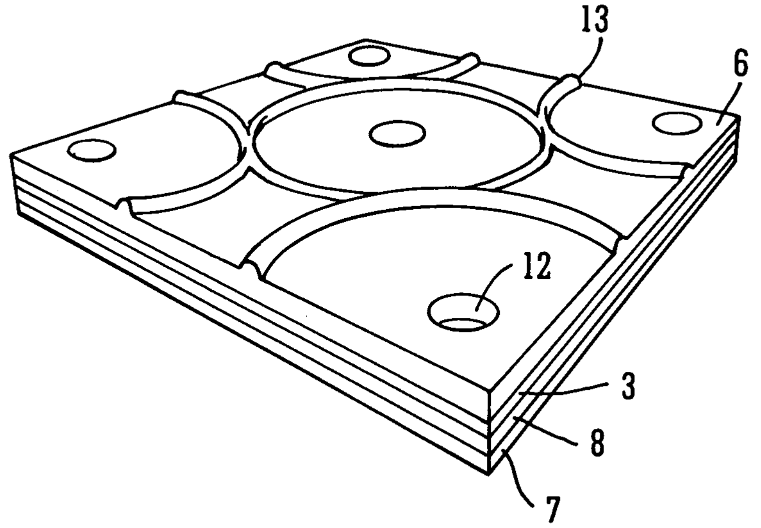
13
6
12
3
8
7

FIG. 2

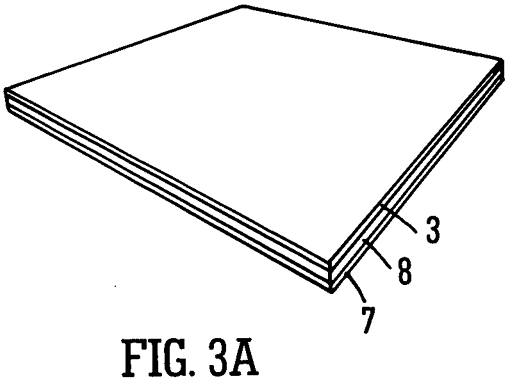

FIG. 3A

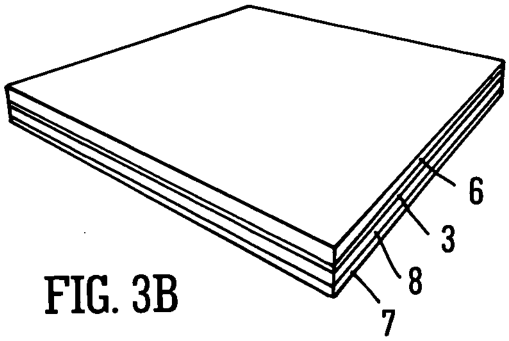

FIG. 3B

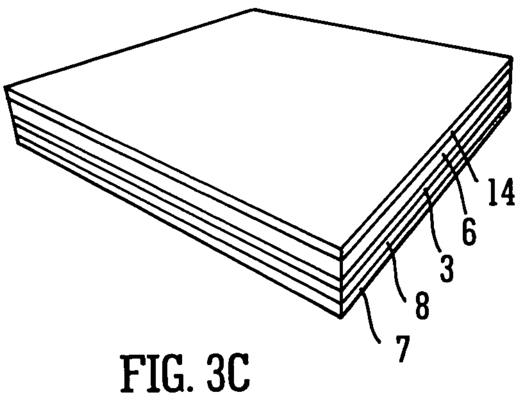

FIG. 3C

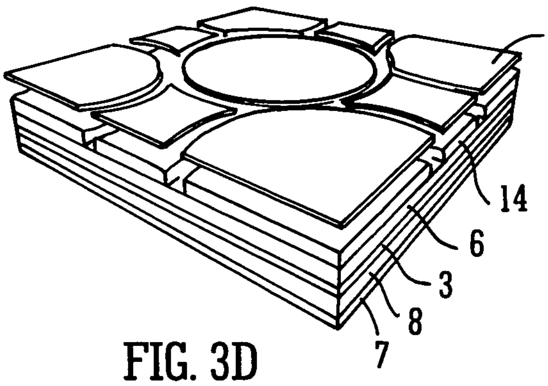

FIG. 3D

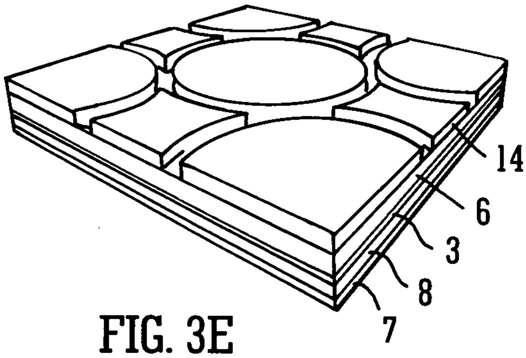

FIG. 3E

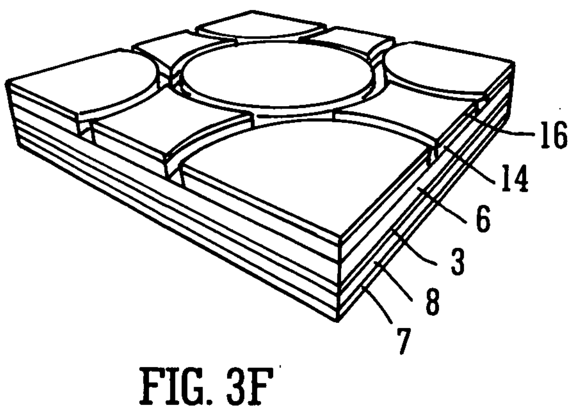

FIG. 3F

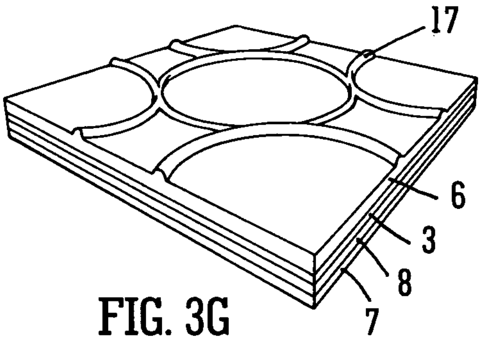


FIG. 3G

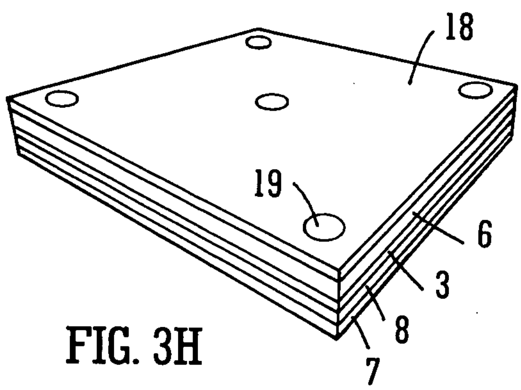


FIG. 3H

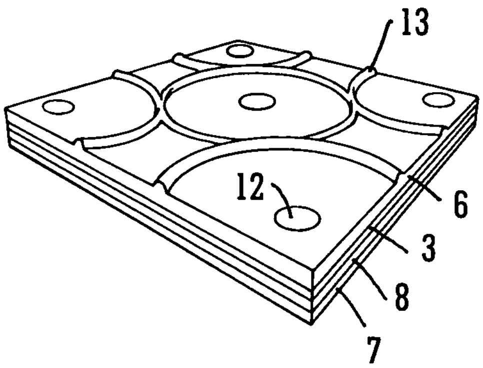


FIG. 3I

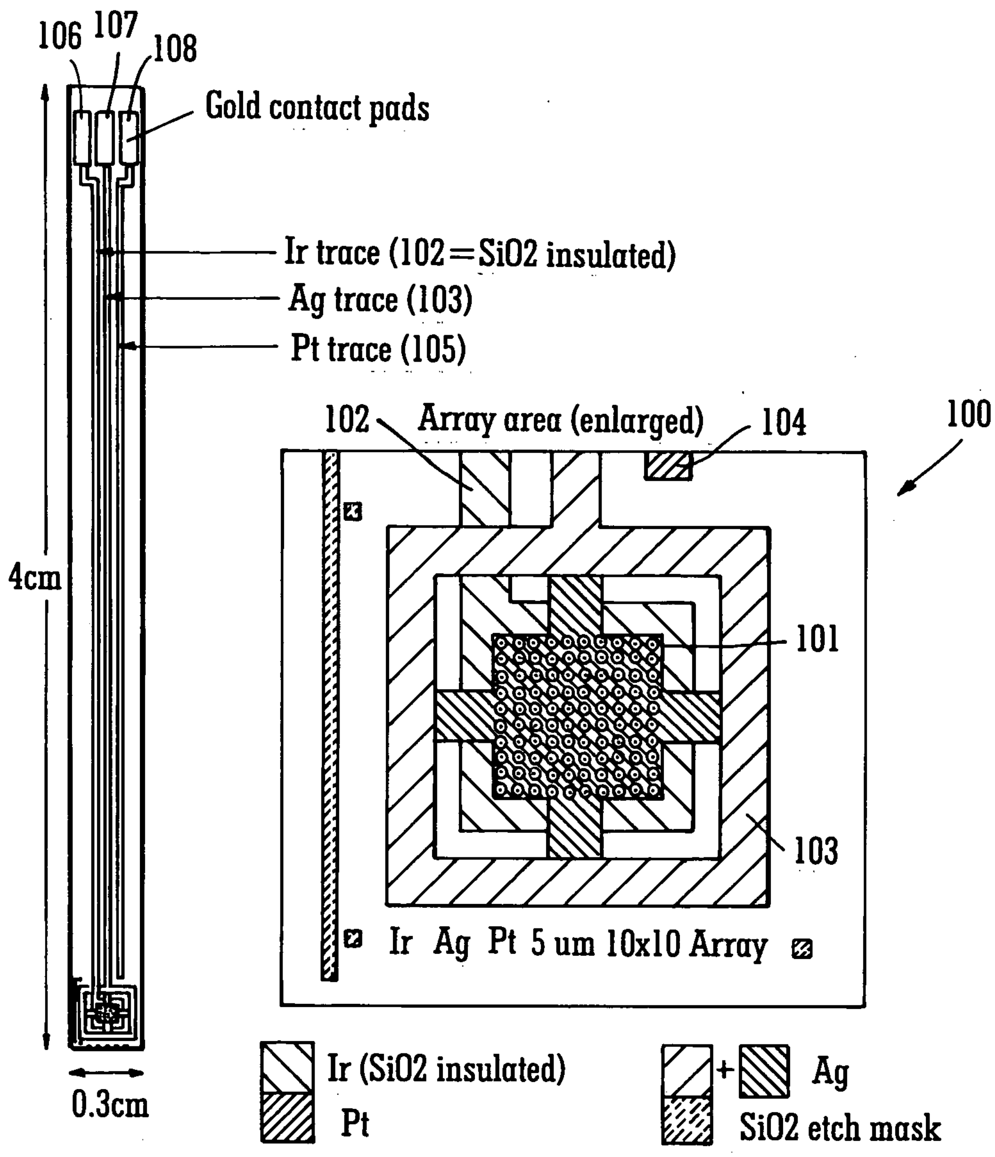
106
107
108
Gold contact pads
Ir trace (102=SiO2 insulated)
Ag trace (103)
Pt trace (105)
4cm
0.3cm
102
Array area (enlarged)
104
100
101
103
Ir Ag Pt 5 um 10x10 Array
Ir (SiO2 insulated)
Pt
+ Ag
SiO2 etch mask

FIG. 4

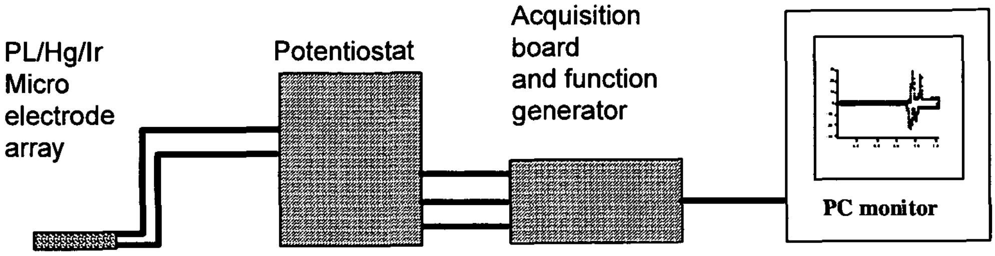
PL/Hg/Ir
Micro
electrode
array
Potentiostat
Acquisition
board
and function
generator
PC monitor

FIG 5

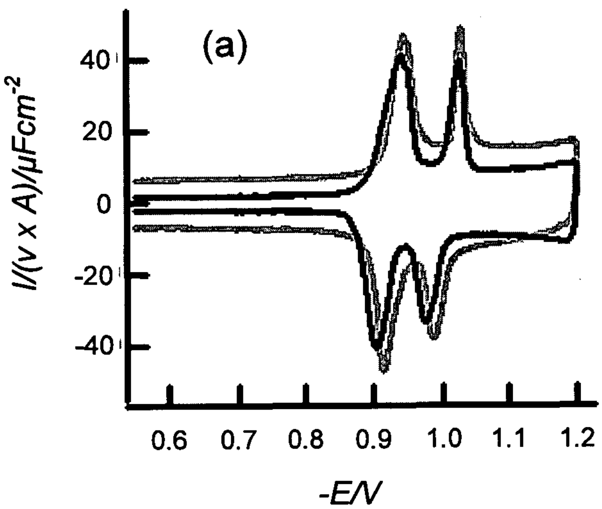
(a)
40
20
0
-20
-40
I/(v x A)/μFcm-2
0.6 0.7 0.8 0.9 1.0 1.1 1.2
-E/V

FIG 6

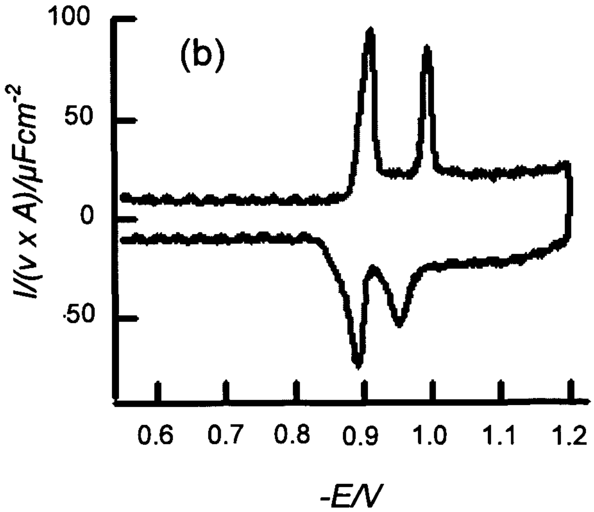
(b)
100
50
0
-50
I/(v x A)/μFcm-2
0.6 0.7 0.8 0.9 1.0 1.1 1.2
-E/V

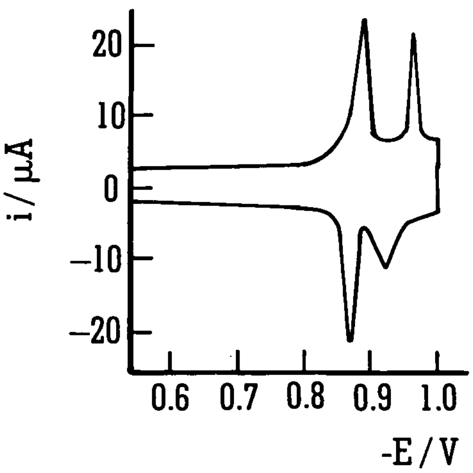
20
10
0
−10
−20
i / μA
0.6
0.7
0.8
0.9
1.0
-E / V

FIG. 7

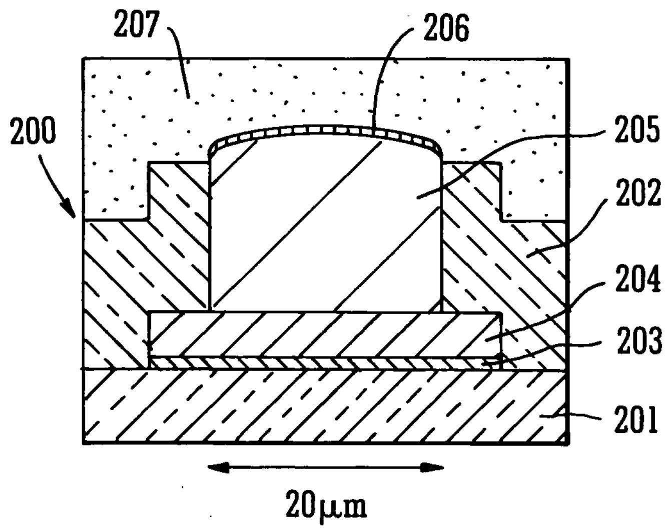
207
206
200
205
202
204
203
201
20μm

FIG. 8

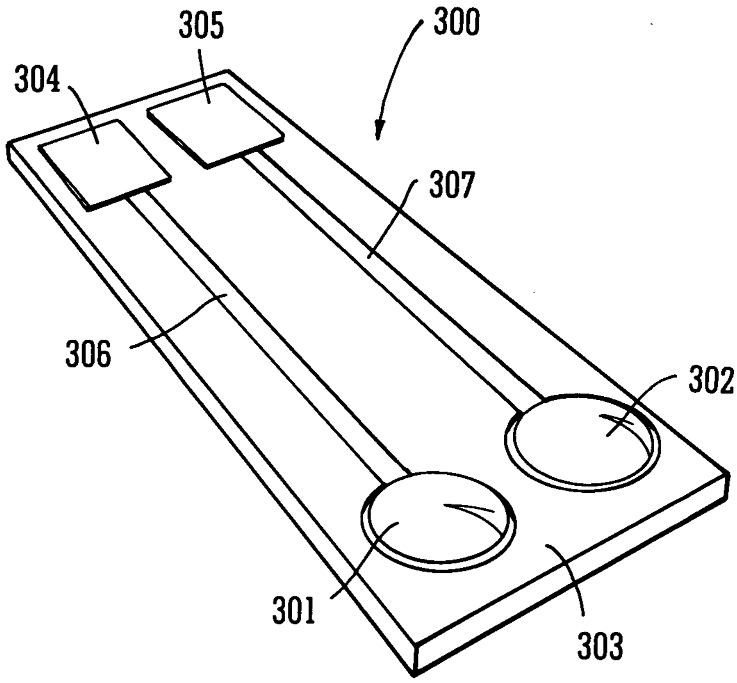


FIG. 9

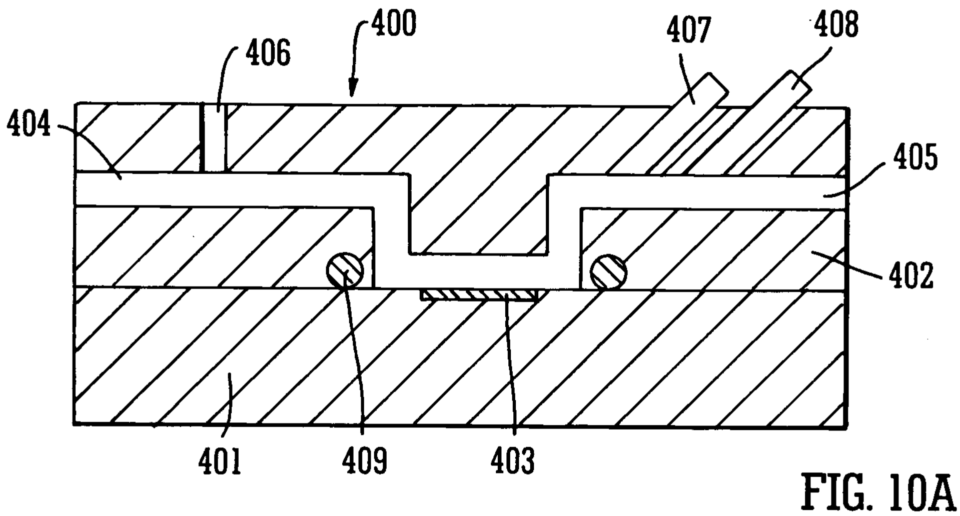


FIG. 10A

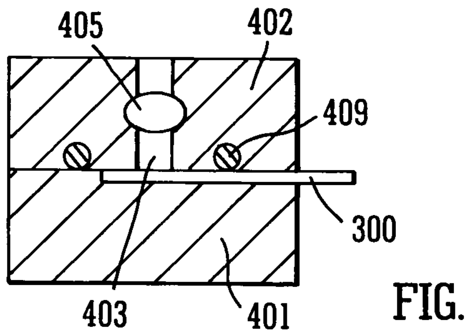


FIG. 10B

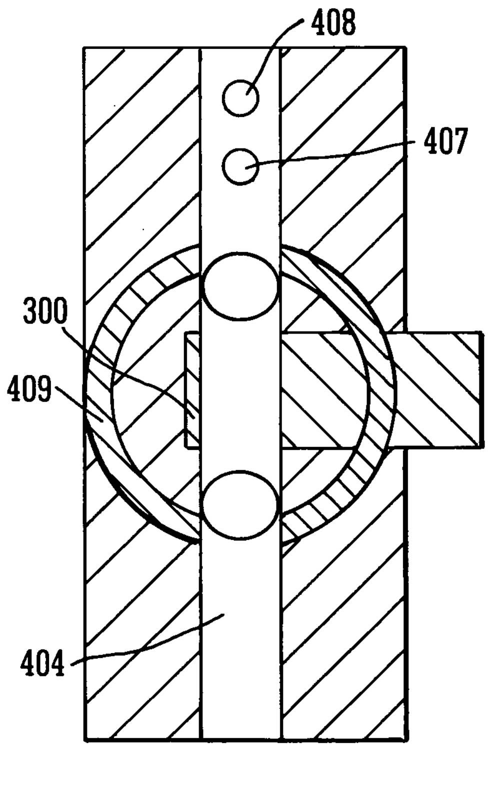


FIG. 10C

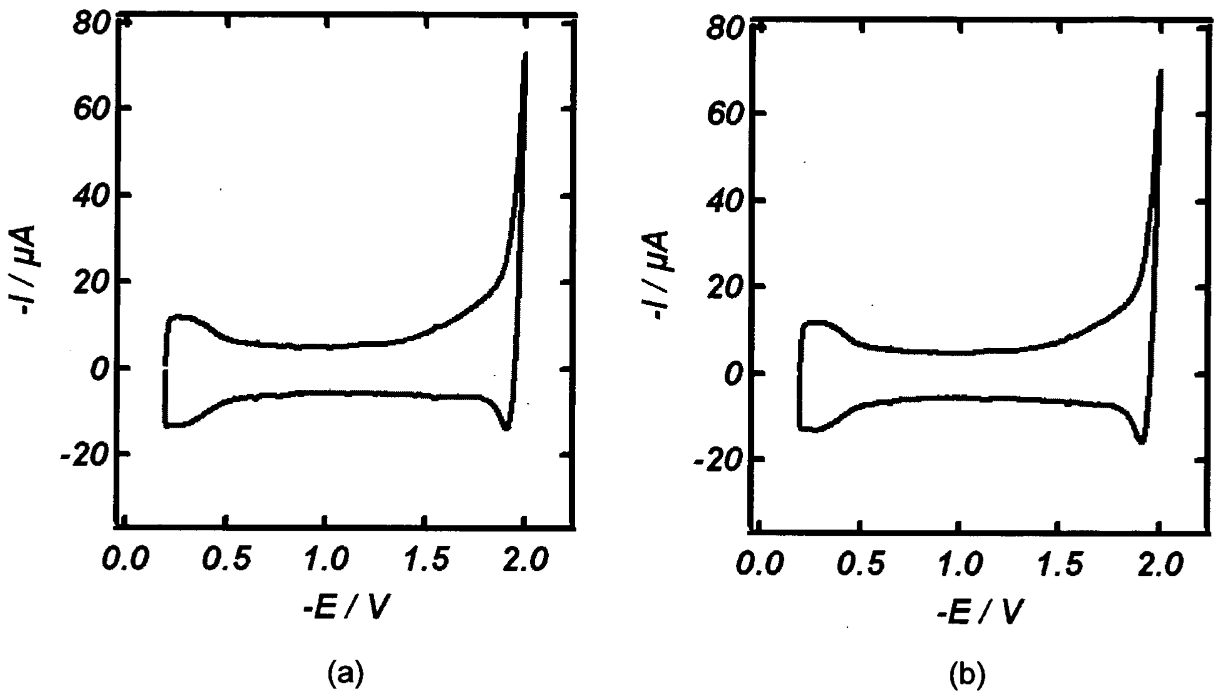

80
60
40
20
0
-20
-I / μA
0.0
0.5
1.0
1.5
2.0
-E / V
(a)
(b)


FIG 11

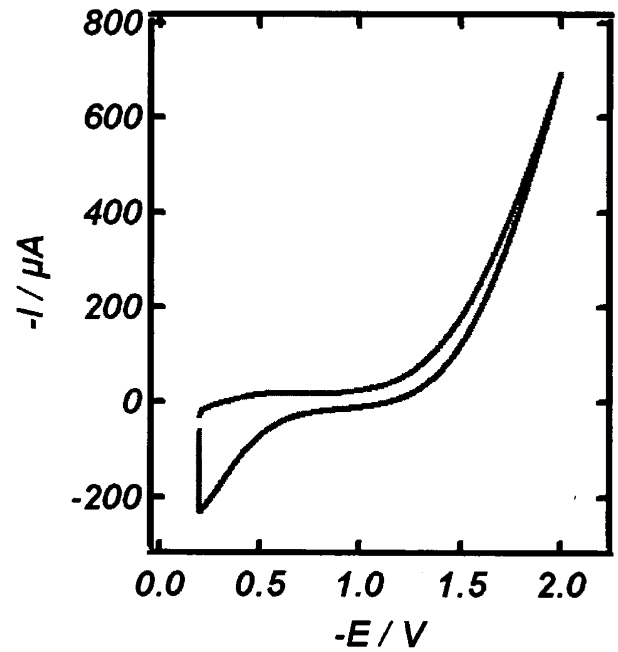

800
600
400
200
0
-200
-I / μA
0.0
0.5
1.0
1.5
2.0
-E / V


FIG 12

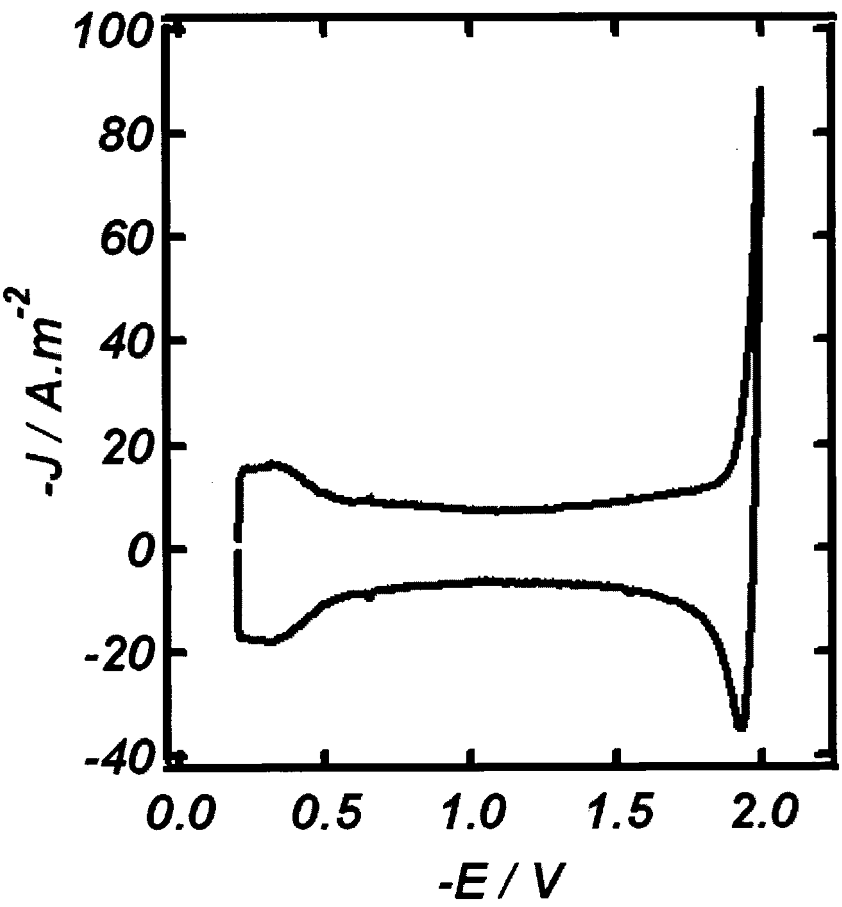


FIG 13

DOPC

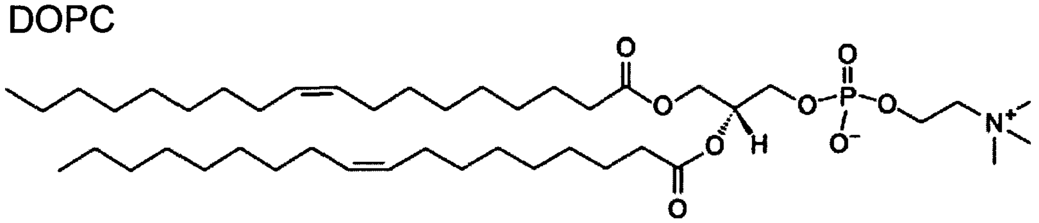

DOPE

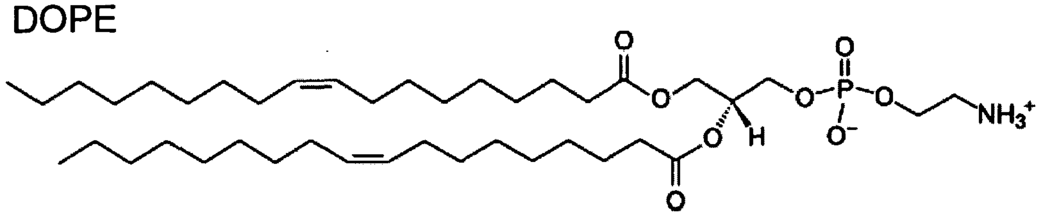

DOPG

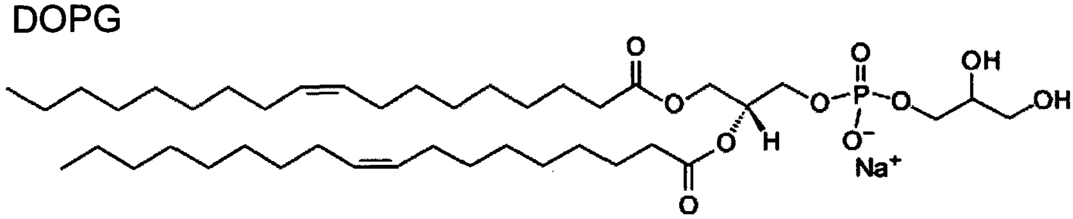

DOPS

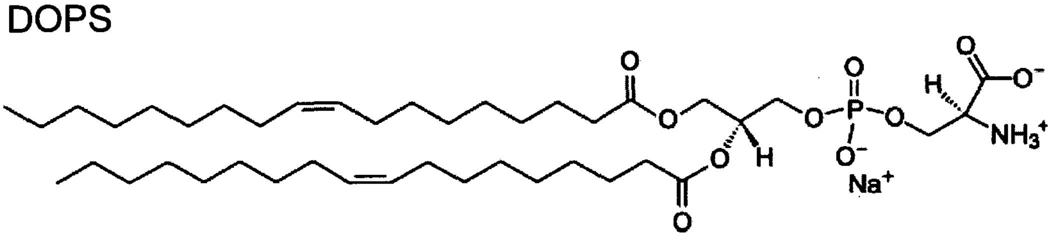

DOPEG

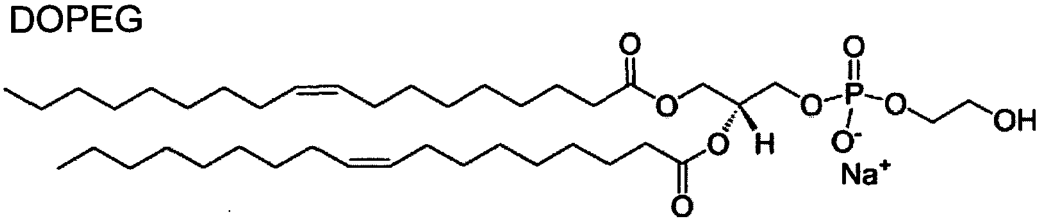

**FIG 14**

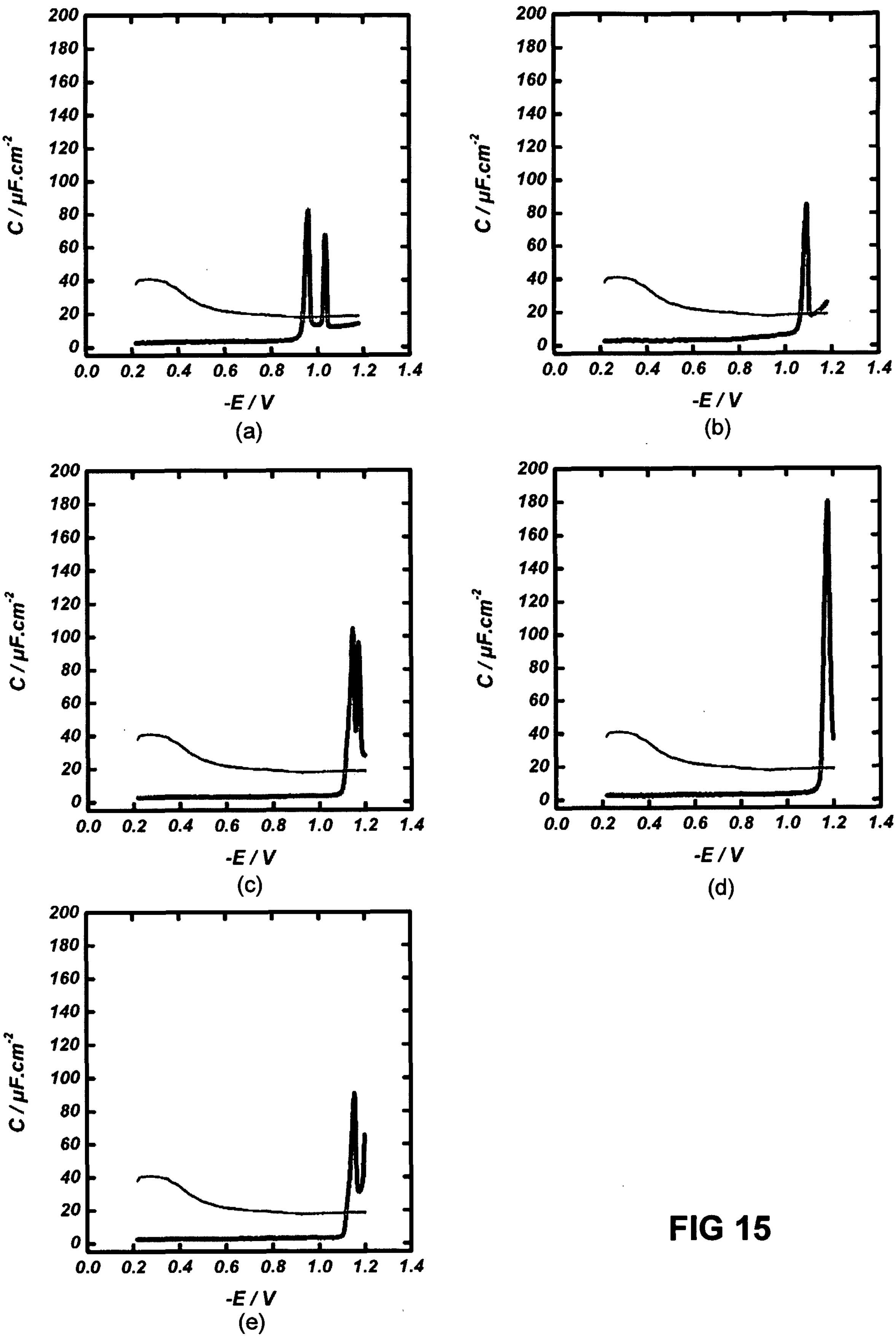


FIG 15

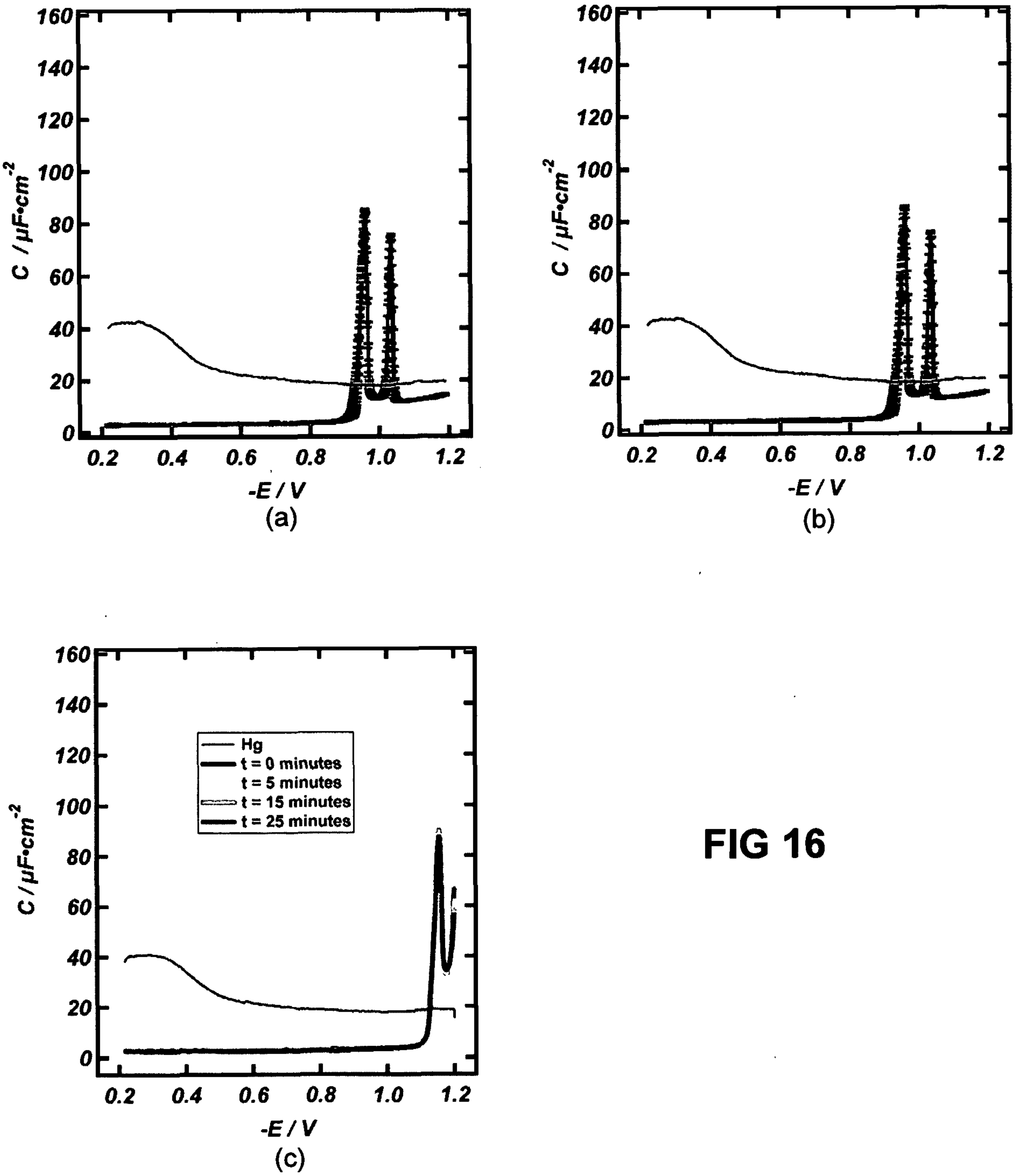
C / μF•cm-2
-E / V
(a)
(b)
(c)
Hg
t = 0 minutes
t = 5 minutes
t = 15 minutes
t = 25 minutes

FIG 16

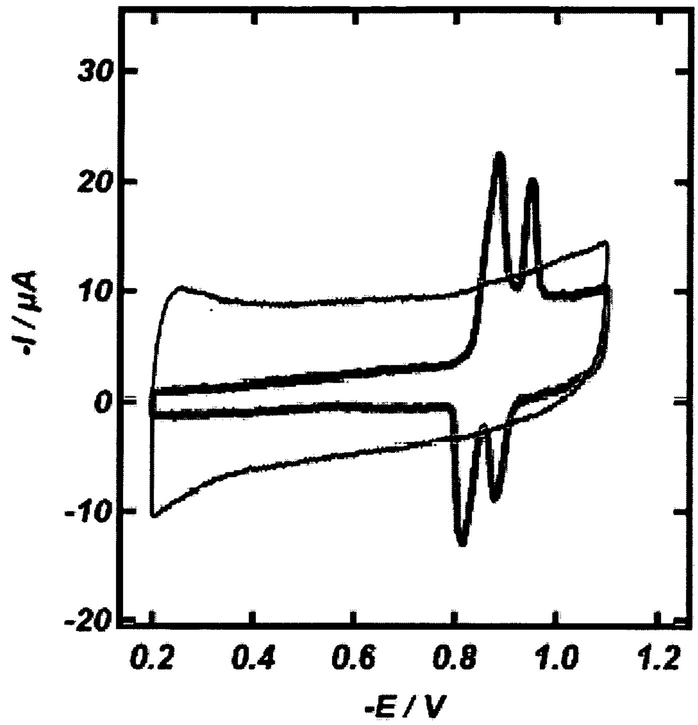
30
20
10
0
-10
-20
-I / μA
0.2
0.4
0.6
0.8
1.0
1.2
-E / V

FIG 17

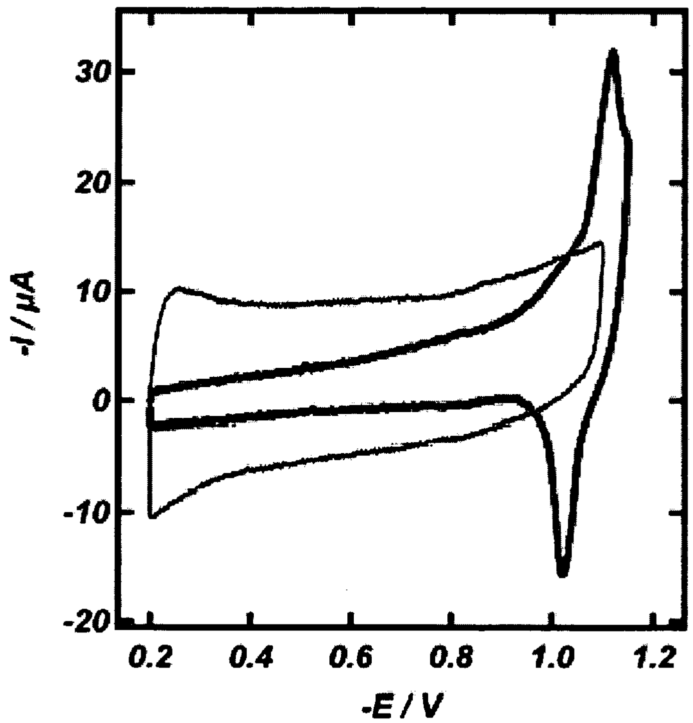
30
20
10
0
-10
-20
-I / μA
0.2
0.4
0.6
0.8
1.0
1.2
-E / V

FIG 18

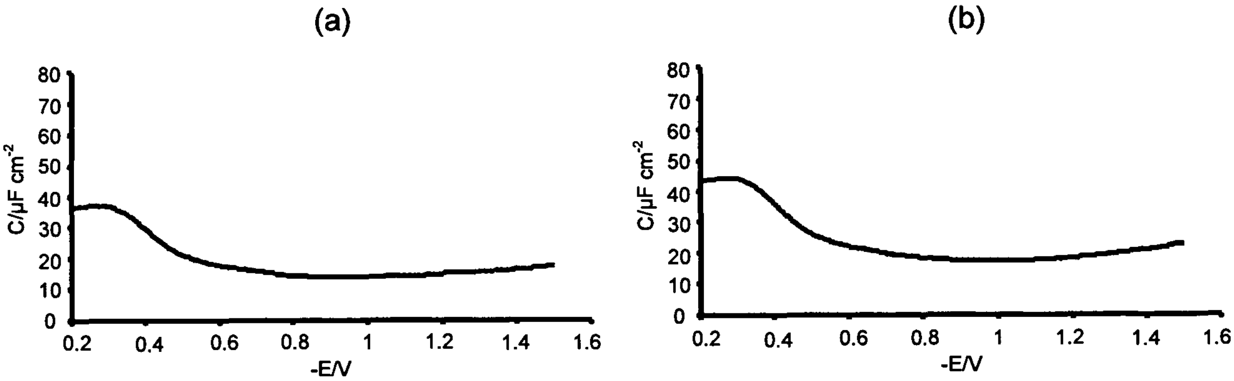
(a)
(b)
C/μF cm-2
-E/V
0 10 20 30 40 50 60 70 80
0.2 0.4 0.6 0.8 1 1.2 1.4 1.6

FIG 19

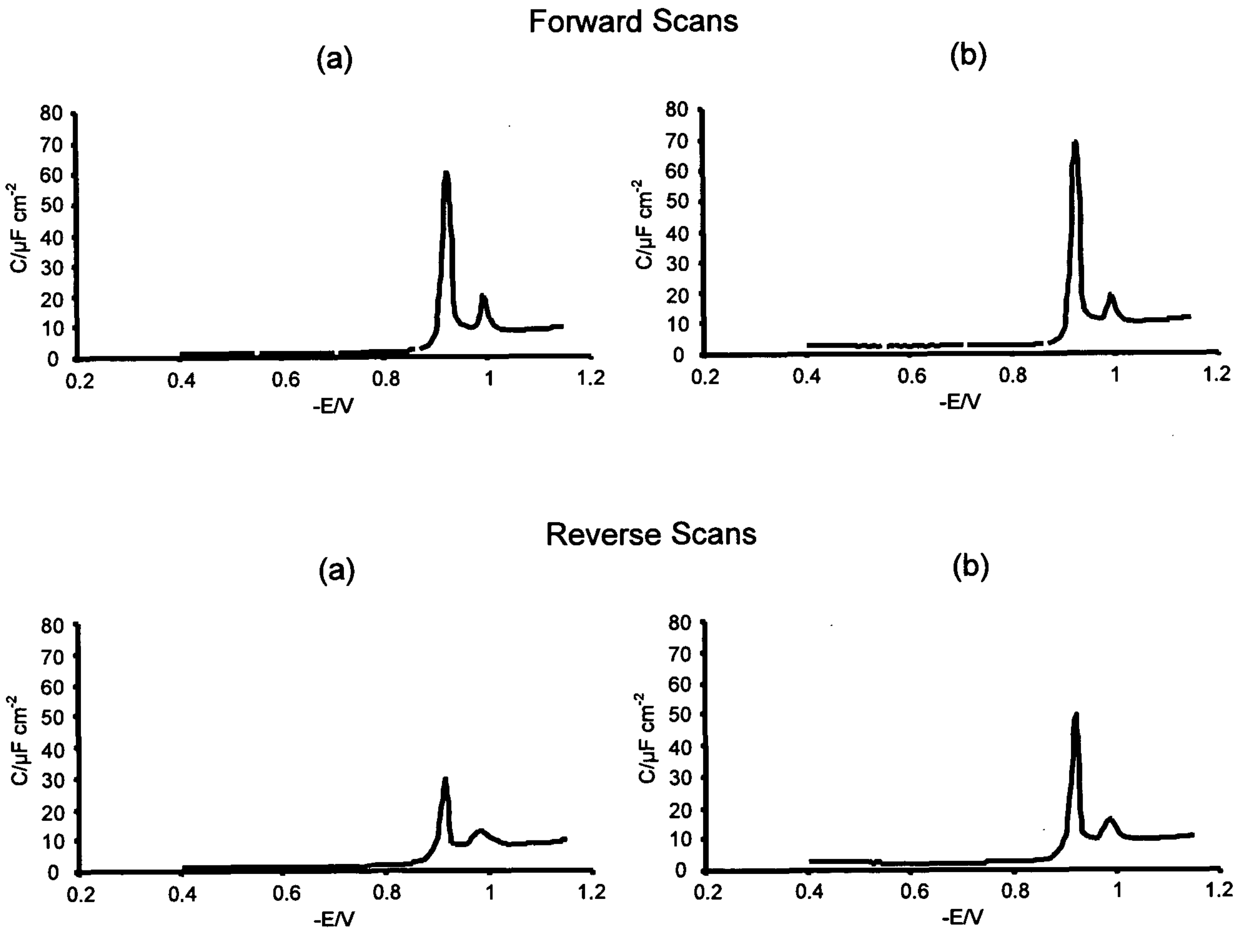
Forward Scans
(a)
(b)
Reverse Scans
(a)
(b)
C/μF cm-2
-E/V
0 10 20 30 40 50 60 70 80
0.2 0.4 0.6 0.8 1 1.2

FIG 20

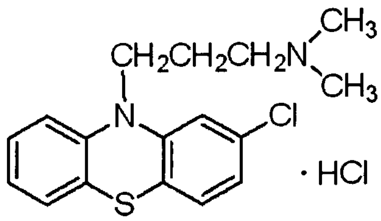

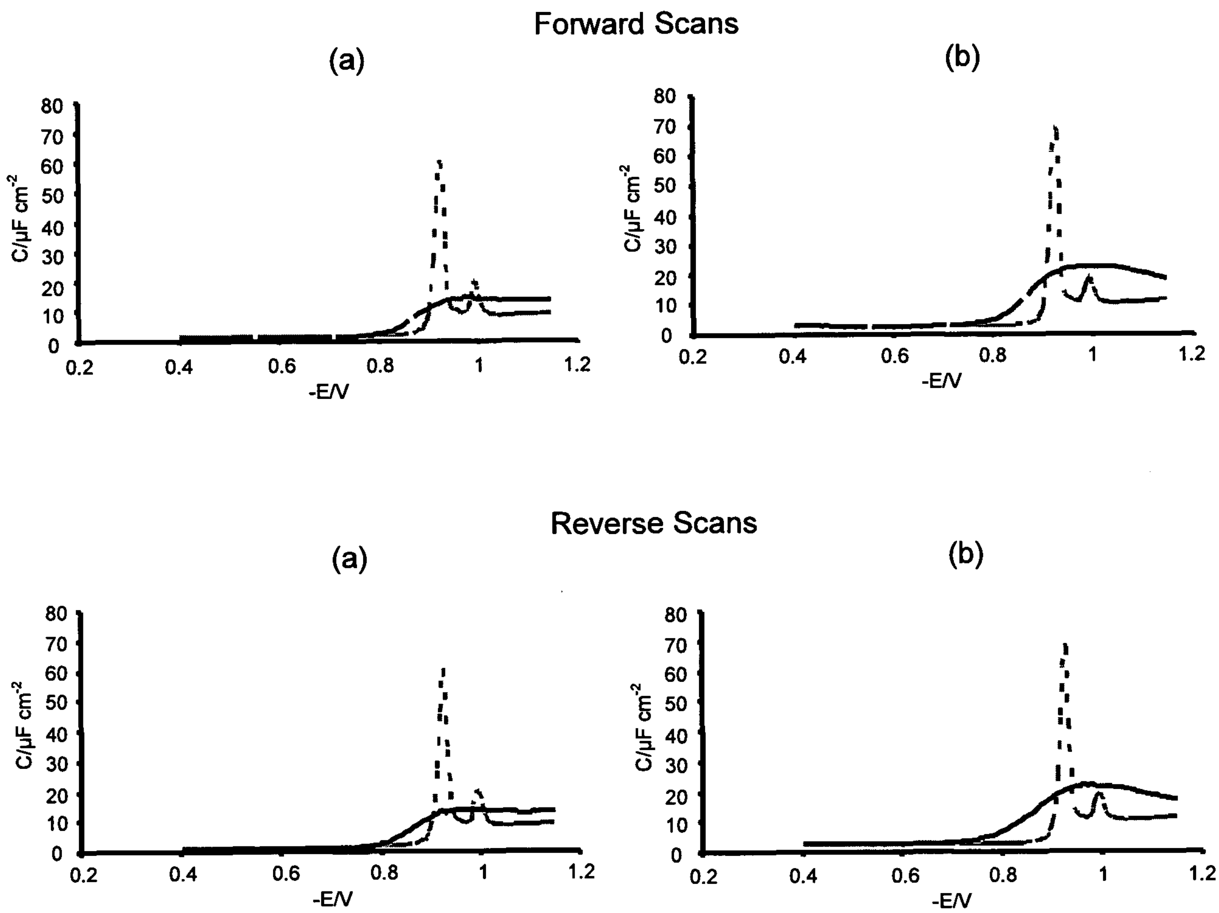


**FIG 21**

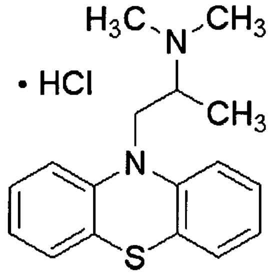

Forward Scans

(a) (b)

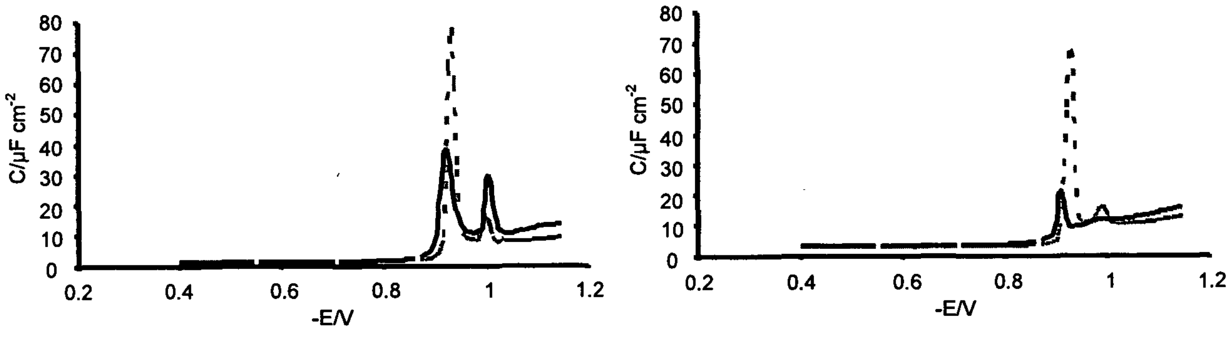


Reverse Scans

(a) (b)

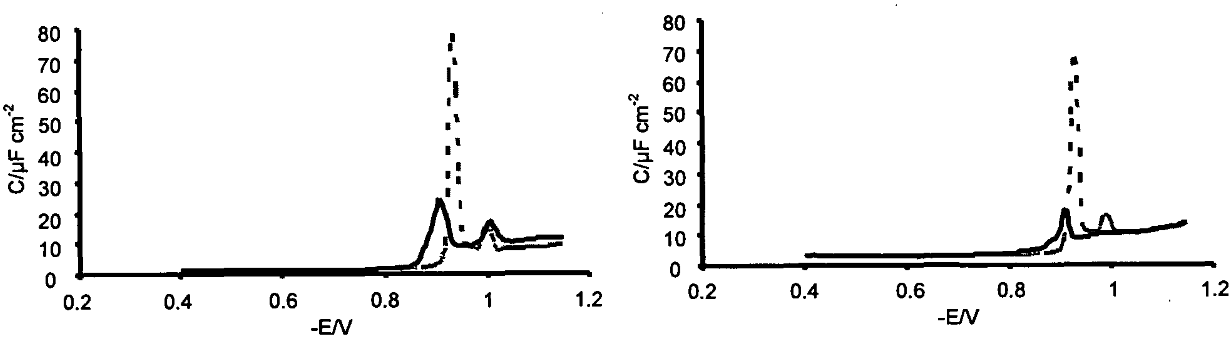


**FIG 22**

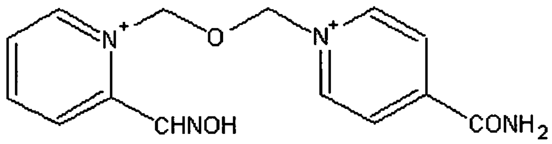

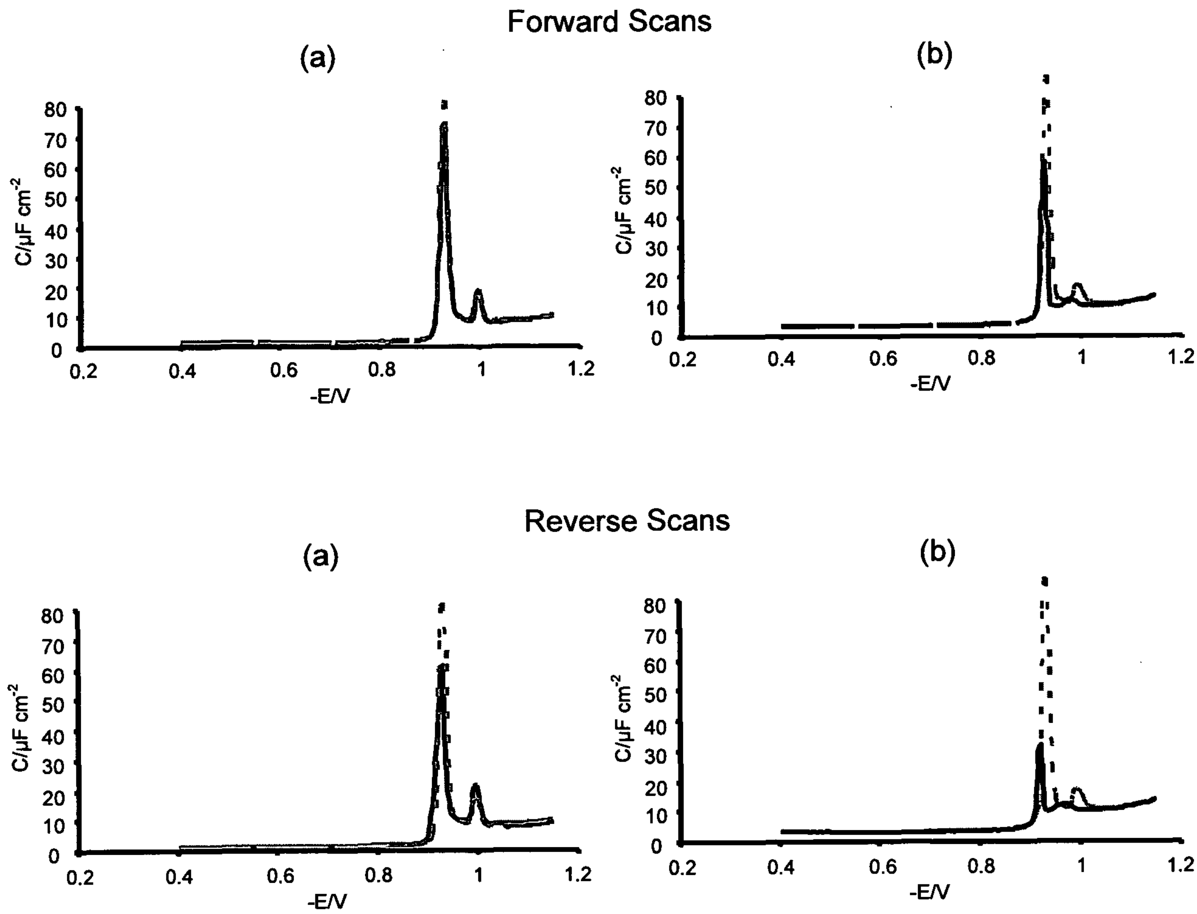
Forward Scans
(a)
(b)
Reverse Scans
(a)
(b)
C/μF cm-2
-E/V
0
10
20
30
40
50
60
70
80
0.2
0.4
0.6
0.8
1
1.2

**FIG 23**

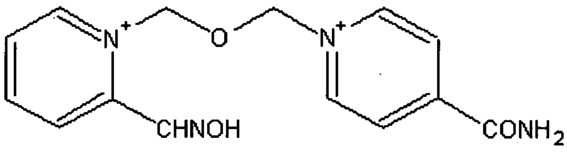

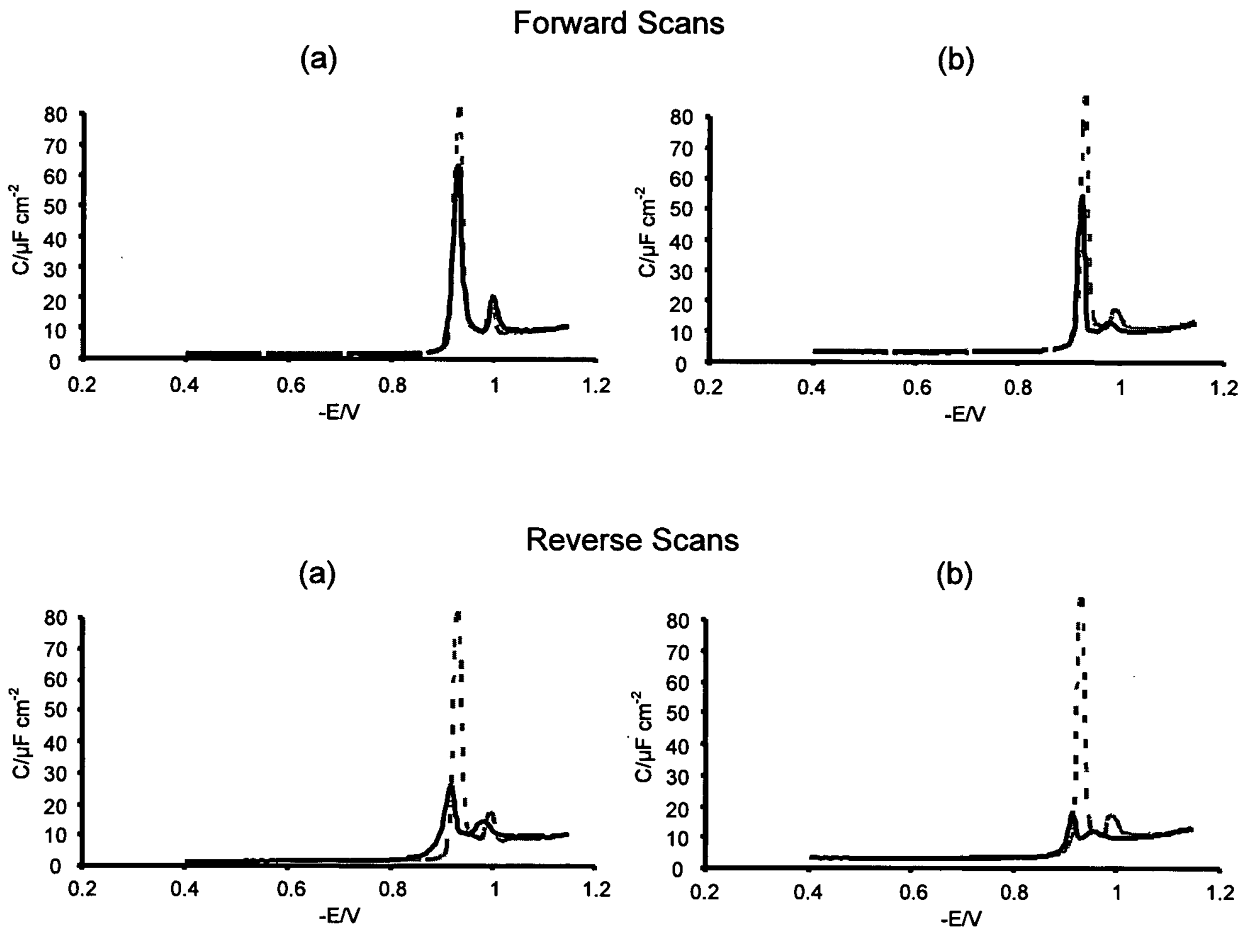
Forward Scans
(a)
(b)
Reverse Scans
(a)
(b)
C/μF cm-2
-E/V
0.2
0.4
0.6
0.8
1
1.2
0
10
20
30
40
50
60
70
80

**FIG 24**

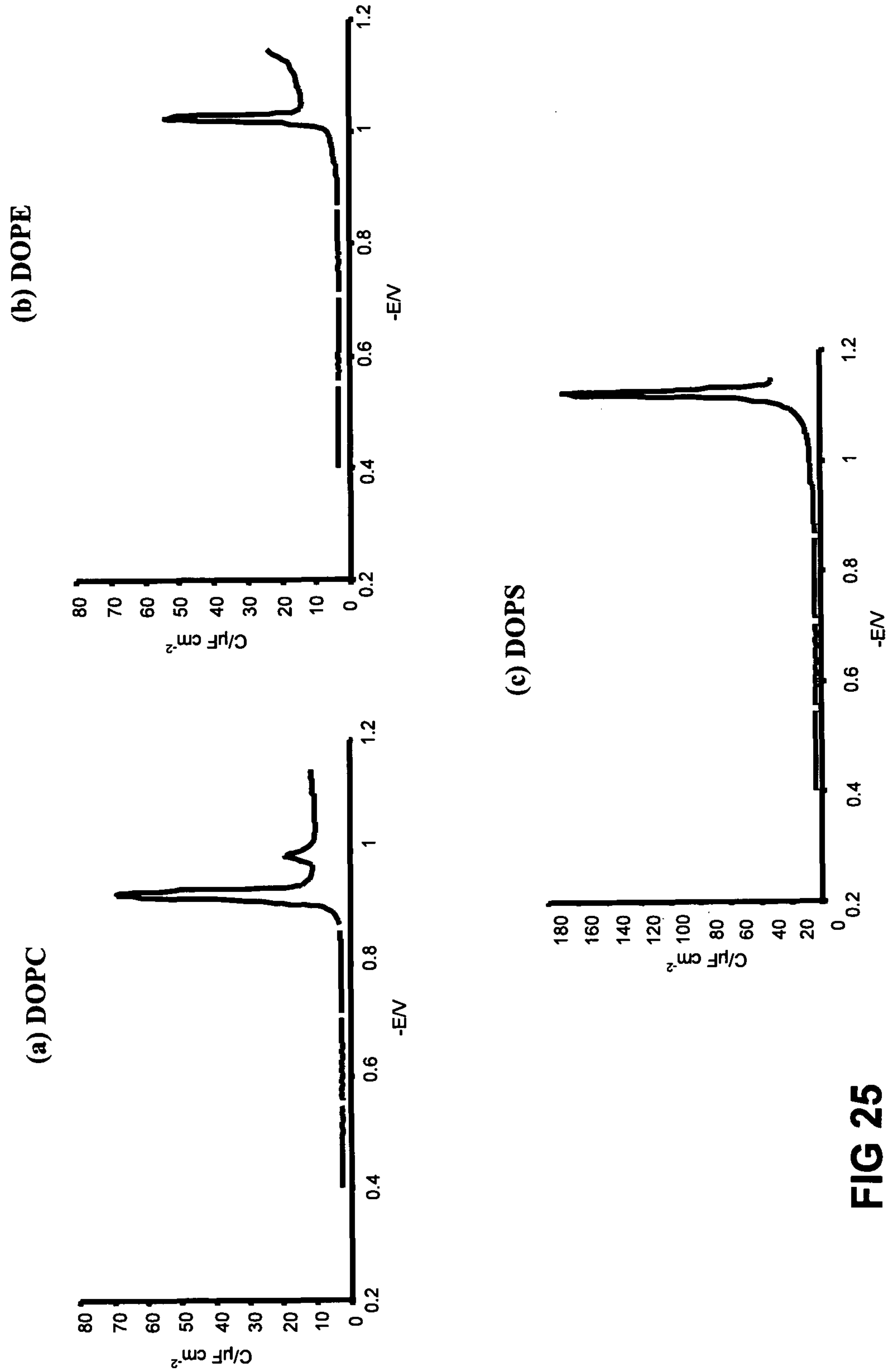
(a) DOPC
(b) DOPE
(c) DOPS
C/μF cm-2
-E/V
0
10
20
30
40
50
60
70
80
100
120
140
160
180
0.2
0.4
0.6
0.8
1
1.2

FIG 25

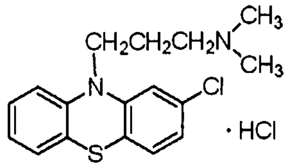

(a) DOPC

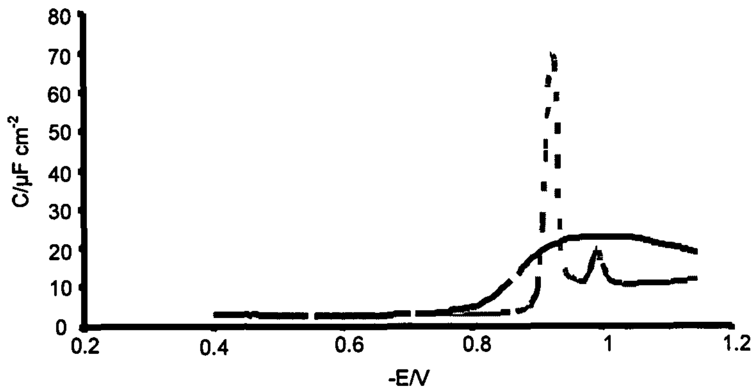


(b) DOPE

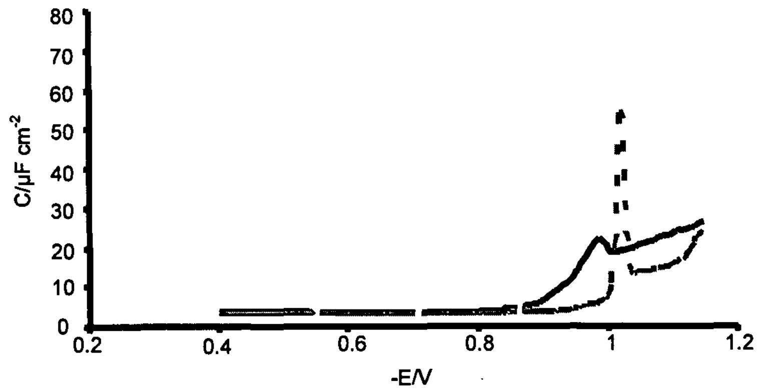


(c) DOPS

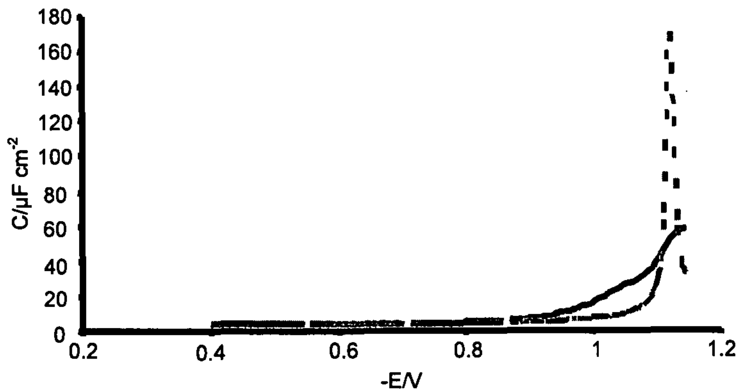


FIG 26

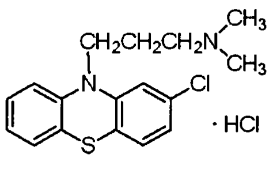

**(b) Chlorpromazine (0.5 μmol dm⁻³)**

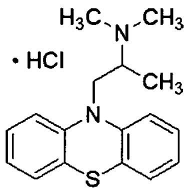

**(b) Promethazine (0.5 μmol dm⁻³)**

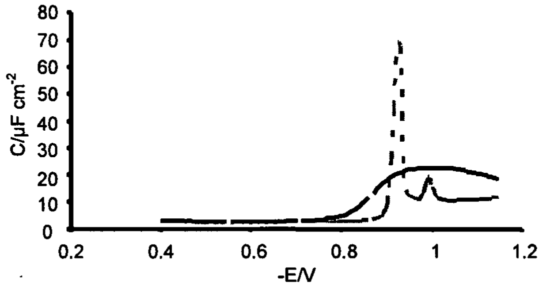


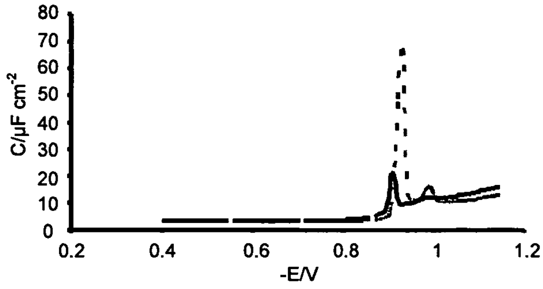


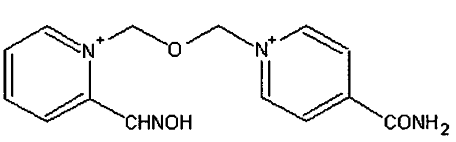

**(c) HI6 (0.5 μmol dm⁻³)**

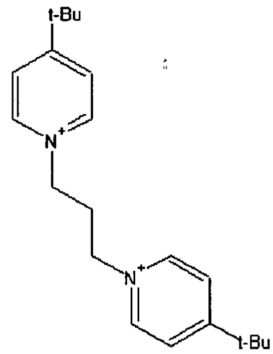

**(d) MB327 (0.5 μmol dm⁻³)**

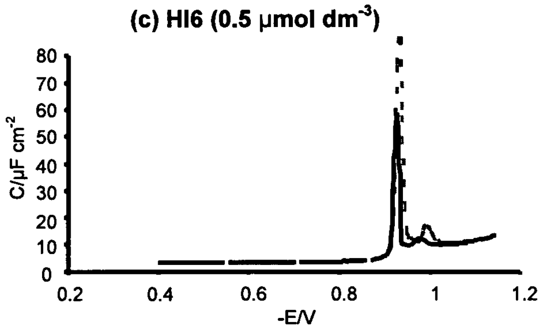


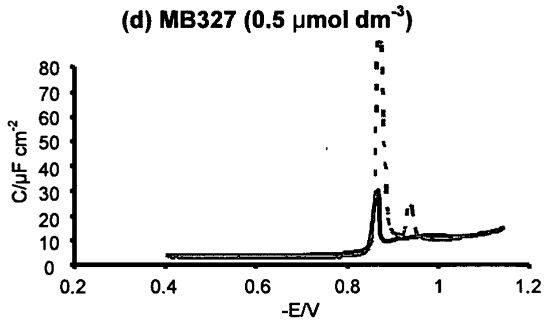


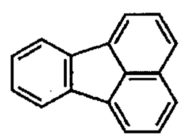

**(e) Fluoranthene (0.5 μmol dm⁻³)**

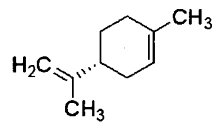

**(f) Limonene (5 μmol dm⁻³)**

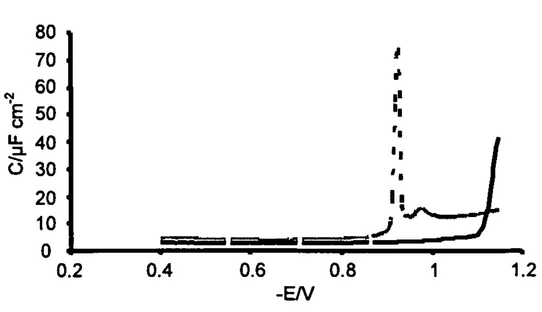


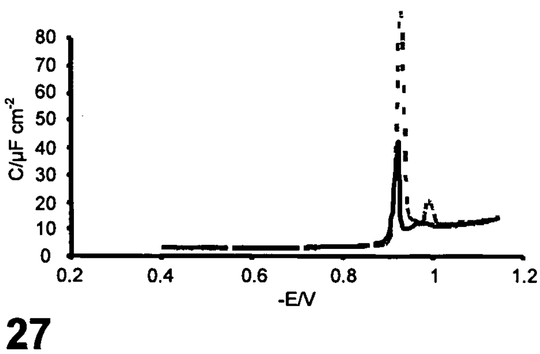


**FIG 27**

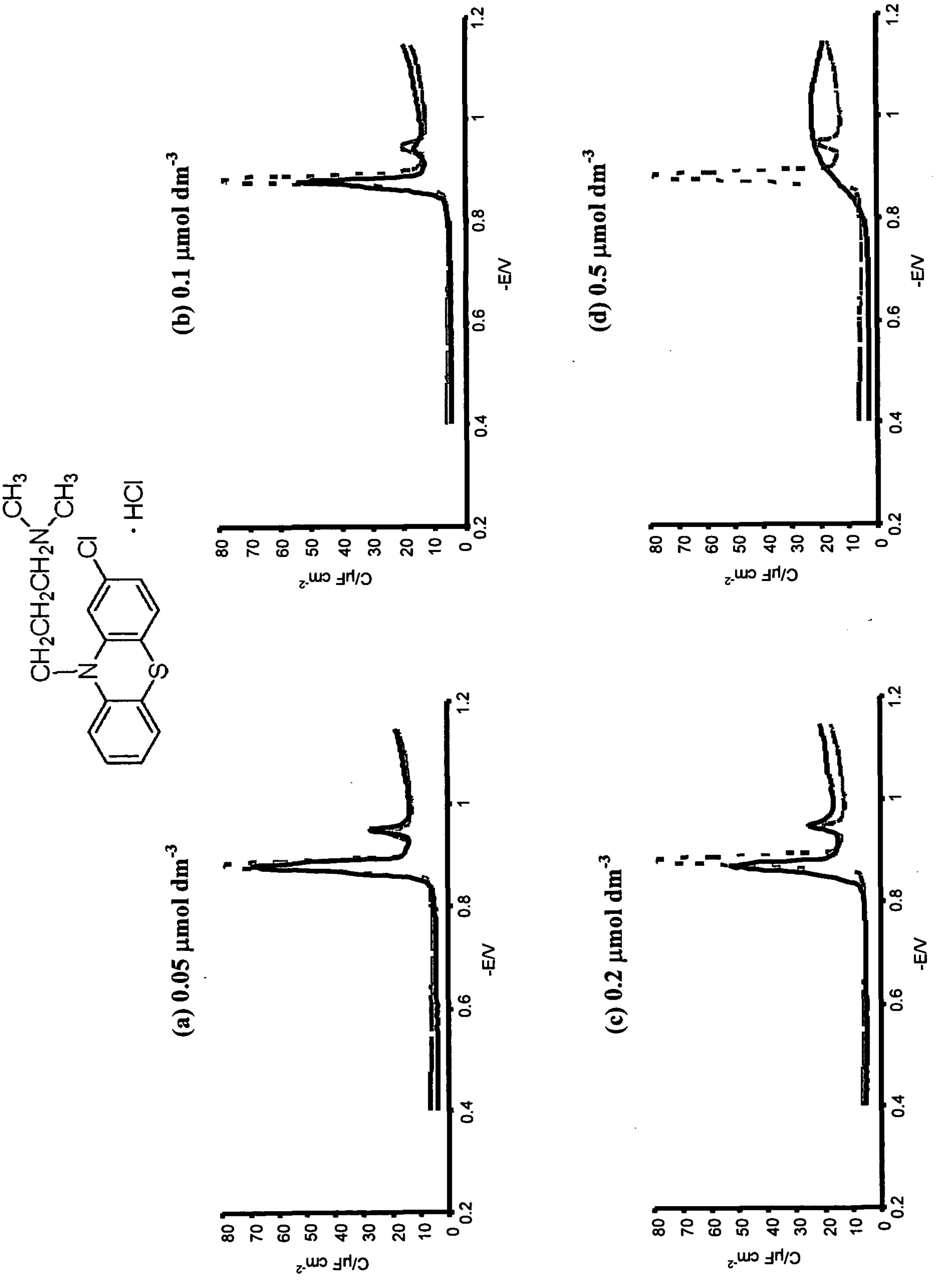
(a) 0.05 µmol dm-3
(b) 0.1 µmol dm-3
(c) 0.2 µmol dm-3
(d) 0.5 µmol dm-3
C/µF cm-2
-E/V
· HCl

FIG 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2193326 A
- US 5378343 A, Kounaves
- GB 2193326 B

### Non-patent literature cited in the description


- *J. Electroanal. Chem.,* 2001, vol. 509, 31-41
- **SACKMANN E.** *Science,* 1996, vol. 271, 43
- **WANUNU, M. ; VASKEVICH, A ; RUBINSTEIN,I.** *J. Am. Chem. Soc.,* 2004, vol. 126, 5569
- **HEYSE, S. ; VOGEL, H. ; SANGER, M. ; SIGRIST, H.** *Protein Sci.,* 1995, vol. 4, 2532
- **SHUMYANTSEVA, V.V. ; IVANOV,Y.D. ; BISTOLAS ,N. ; SCHELLER, F.W. ; ARCHAKOV, A.I. ; WOLLENBERGER, U.** *Anal.Chem.,* 2004, vol. 76, 6046
- **BECUCCI, L. ; LEON, R.R. ; MONCELLI, MR ; ROVERO, P. ; GUIDELLI, R.** *Langmuir,* 2006, vol. 22, 6644
- **NELSON.A.** *Anal. Chim. Acta,* 1987, vol. 194, 139
- **NELSON, A. ; AUFFRET,N. ; READMAN,J.** *Anal. Chim. Acta,* 1988, vol. 207, 45
- **NELSON,A. ; AUFFRET,N. ; BORLAKOGLU,J.** *Biochim. Biophys. Acta,* 1990, vol. 1021, 205
- **BIZZOTTO, D ; NELSON, A.** *Langmuir,* 1998, vol. 14, 6269
- **MONNE J ; GALCERAN J ; PUY J ; NELSON A.** *Langmuir,* 2003, vol. 19, 4694
- **WHITEHOUSE, C ; O'FLANAGAN, R ; LINDHOLM-SETHSON, B ; MOVAGHAR, B ; NELSON, A.** *Langmuir,* 2004, vol. 20, 136
- **WHITEHOUSE, C ; GIDALEVITZ, D ; CAHUZAC, M ; KOEPPE, R.E II ; NELSON, A.** *Langmuir,* 2004, vol. 20, 9291
- **NELSON, A.** *Biophys. J.,* 2001, vol. 80, 2694
- **PROTOPAPA,E.P. ; AGGELI,A. ; BODEN,N. ; KNOWLES,P.F. ; SALAY,L.C. ; NELSON,A.** *Medical Engineering and Physics,* 2006, vol. 28, 944
- **MERRIFIELD,J. ; TATTERSALL,J.E.H. ; BIRD,M. ; NELSON,A.** *Electroanalysis*
- **WEISS,S ; MILLNER,P. ; NELSON,A.** *Electrochimica Acta,* 2005, vol. 50, 4248
- **BURGERS,I ; LI,M. ; HORSWELL,S.L. ; SZYMANSKI,G. ; LIPKOWSKI,J. ; SATIJA,S. ; MAJEWSKI, J.** *Colloids and Surfaces B-Biointerfaces,* 2005, vol. 40, 117
- **BIN,X.M. ; ZAWISZA,I. ; GODDARD, J.D. ; LIPKOWSKI,J.** *Langmuir,* 2005, vol. 21, 330
- **NELSON, A.** *J.Electroanal.Chem.,* 2006
- **WILLMANN, S. et al.** *J. Med. Chem.,* 2004, vol. 47, 4022
- **F. NEVILLE ; M. CAHUZAC ; A. NELSON ; D. GIDALEVITZ.** *Journal of Physics-Condensed Matter,* 2004, vol. 16 (26), S2413-S2420
- **F. NEVILLE ; D. GIDALEVITZ ; G. KALE ; A. NELSON.** *Bioelectrochemistry,* 2006
- **STOODLEY,R. ; BIZZOTTO,D.** *Analyst,* 2003, vol. 128, 552
- **KOUNAVES, S.P. ; DENG, W.** *Anal. Chem.,* 1993, vol. 65, 375
- **NOLAN, M.A. ; KOUNAVES, S.P.** *Anal. Chem.,* 1999, vol. 71, 3567
- **D. C. GRAHAME.** *Journal Of The American Chemical Society,* 1949, vol. 71 (9), 2975-2978